# EUROPEAN PATENT APPLICATION

(11) **EP 4 763 061 A1**
(43) Date of publication of application: **24.06.2026**
(21) Application number: 24868474.8
(22) Date of filing: 07.08.2024
(51) Int. Cl.: A61B 5/01, A61B 5/00, A61B 8/00

(54) **WEARABLE DEVICE AND BODY TEMPERATURE MEASURING METHOD USING ULTRASONIC WAVES**

(30) Priority: 19.09.2023 KR 20230124683; 07.11.2023 KR 20230152440
(71) Applicant: Samsung Electronics Co., Ltd., Suwon-si, Gyeonggi-do 16677 (KR)
(72) Inventor: KANG, Kichang, Suwon-si, Gyeonggi-do 16677 (KR); KIM, Suna, Suwon-si, Gyeonggi-do 16677 (KR); SANG, Pilgyu, Suwon-si, Gyeonggi-do 16677 (KR)
(74) Representative: Gulde & Partner
(86) International application number: PCT/KR2024/011730
(87) International publication number: WO 2025/063495

(57) **Abstract**

A body temperature measuring method using ultrasonic waves, of an electronic device, comprises: setting a reference body temperature on the basis of at least one among body temperature information measured through a temperature sensor or body temperature information received from an external device; in a state where the electronic device is worn by a user, measuring a first speed of an ultrasonic wave passing through the body of the user, by using at least one ultrasonic sensor; storing the measured first speed of the ultrasonic wave and the set reference body temperature as reference information in a memory; after storing the reference information, measuring a second speed of an ultrasonic signal passing through the body of the user, by using the at least one ultrasonic sensor; and generating compensated body temperature information by compensating for the reference body temperature on the basis of the difference between the measured first speed and second speed.

## Description

### [Technical Field]

The disclosure relates to a wearable device and, more particularly, to a method for measuring the body temperature of a user by using an ultrasonic sensor included in the wearable device.

### [Background Art]

Various types of electronic devices are being developed to provide diverse user experiences. A wearable device is an electronic device that can be worn on the user's body. For example, such a wearable device may be configured in a form that can be worn on the user's head, wrist, or finger, and provide a variety of functions. As an example of a wearable device, a smart ring that can be worn on the finger in the form of a ring is being developed.

A wearable device such as a smart ring may include biometric sensors that, when worn on the user's body, can measure various biometric information such as body temperature, heart rate, electrocardiogram, and blood pressure. A temperature sensor that measures body temperature can measure the temperature of a user's skin through an optical signal (e.g., infrared signal) based on the Stefan-Boltzmann law, which states that radiant energy per unit area is proportional to the fourth power of the absolute temperature.

The above information may be provided as related art to aid in understanding the disclosure. No claim or determination is made as to whether any of the above-described matters constitute prior art in relation to the disclosure.

### [Disclosure of Invention]

### [Technical Problem]

When measuring body temperature using a temperature sensor utilizing an optical signal in a wearable device such as a smart ring, accuracy may be reduced according to external factors because the temperature is measured at the skin surface. For example, due to the nature of a wearable device worn on the wrist or finger, it may be easily affected by the clothes or gloves the user is wearing, and there may be an error compared to the actual body temperature because the skin is exposed to the external temperature.

### [Solution to Problem]

An electronic device according to the disclosure (or, specification, invention) may include a temperature sensor for measuring the user's body temperature, a wireless communication circuit for supporting wireless communication with an external device, at least one ultrasonic sensor, a memory, and at least one processor operably connected to the temperature sensor, the wireless communication circuit, the at least one ultrasonic sensor, and the memory.

According to an embodiment, the memory may store instructions that are executable by the at least one processor and, when executed, cause the electronic device to: set a reference body temperature based on at least one of body temperature information measured by the temperature sensor or body temperature information received through the wireless communication circuit from the external device; measure a first velocity of ultrasonic waves passing through the user's body by using the at least one ultrasonic sensor while the electronic device is worn by the user; and store the measured first velocity of ultrasonic waves and the set reference body temperature in the memory as reference information.

According to an embodiment, the memory may store instructions that cause the electronic device to: measure, after storing the reference information, a second velocity of ultrasonic signals passing through the user's body by using the at least one ultrasonic sensor; and generate compensated body temperature information by compensating for the reference body temperature based on a difference between the measured first velocity and the second velocity.

A method for an electronic device to measure the body temperature using ultrasonic waves according to various embodiments of this document may include: setting a reference body temperature based on at least one of body temperature information measured by a temperature sensor or body temperature information received from an external device; measuring a first velocity of ultrasonic waves passing through the user's body by using at least one ultrasonic sensor while the electronic device is worn by the user; storing the measured first velocity of ultrasonic waves and the set reference body temperature in a memory as reference information; measuring, after storing the reference information, a second velocity of ultrasonic signals passing through the user's body by using the at least one ultrasonic sensor; and generating compensated body temperature information by compensating for the reference body temperature based on a difference between the measured first velocity and the second velocity.

An electronic device according to various embodiments of this document may include a display, a wireless communication circuit for supporting wireless communication with an external device, a memory, and at least one processor operably connected to the display, the wireless communication circuit, and the memory, wherein the memory may store instructions that are executable by the at least one processor and, when executed, cause the electronic device to: set a reference body temperature based on at least one of a user input through the display or body temperature information received through the wireless communication circuit from the external device; receive, through the wireless communication circuit, a first velocity of ultrasonic waves passing through the user's body, measured by the external device using at least one ultrasonic sensor; store the measured first speed of ultrasonic waves and the set reference body temperature as reference information in the memory; receive, after storing the reference information, through the wireless communication circuit, a second velocity of ultrasonic waves passing through the user's body, measured by the external device using the at least one ultrasonic sensor; and generate compensated body temperature information by compensating for the reference body temperature based on a difference between the received first velocity and the second velocity.

An electronic device according to various embodiments of this document may include a first ultrasonic sensor, a second ultrasonic sensor disposed to be spaced apart from the first ultrasonic sensor, a memory, and a processor operably connected to the first ultrasonic sensor, the second ultrasonic sensor, and the memory, wherein the memory may store a mapping table that maps the movement velocity of an ultrasonic signal and the user's body temperature for each movement path of the ultrasonic signal, and wherein the memory may store instructions that are executable by at least one processor and, when executed, cause the electronic device to: identify the movement velocity of a first ultrasonic signal that is output from the first ultrasonic sensor and received through a direct path between the first ultrasonic sensor and the second ultrasonic sensor; identify the movement velocity of a second ultrasonic signal that is output from the first ultrasonic sensor, reflected by a part of the user's body, and received through a reflected path; calculate a weighted average of the movement velocity of the first ultrasonic signal and the movement velocity of the second ultrasonic signal; obtain, from the mapping table stored in the memory, body temperature information corresponding to the weighted average of the movement velocity of the first ultrasonic signal and the movement velocity of the second ultrasonic signal, and determine the body temperature information obtained from the mapping table to be the user's current body temperature.

### [Advantageous Effects of Invention]

According to various embodiments of the present document, it is possible to provide a body temperature measurement method using ultrasonic waves, which can measure the temperature inside the user's body using an ultrasonic sensor, and which can measure the body temperature more accurately by using ultrasonic signals of various paths to improve the accuracy of the ultrasonic sensor.

### [Brief Description of Drawings]

FIG. 1 is a block diagram illustrating an electronic device 101 in a network environment 100 according to various embodiments.
FIG. 2 illustrates a wearable device and a portable device according to an embodiment.
FIG. 3A is a block diagram of the wearable device according to an embodiment.
FIG. 3B is a diagram illustrating a sensor arrangement structure of the wearable device according to an embodiment.
FIG. 4 is a block diagram of the portable device according to an embodiment.
FIG. 5 illustrates the principle of the ultrasonic sensor in the wearable device according to an embodiment.
FIG. 6 is a graph showing the relationship between the temperature of a medium and the velocity of ultrasonic waves according to an embodiment.
FIG. 7 is a diagram for explaining changes in the velocity of an ultrasonic signal with changes in body temperature according to an embodiment.
FIG. 8 illustrates a signal detected at the receiving end of an ultrasonic sensor according to an embodiment.
FIG. 9 illustrates signals measured on the direct path and reflected path of ultrasonic signals according to an embodiment.
FIG. 10 illustrates transmission paths of ultrasonic signals according to an embodiment.
FIG. 11 illustrates changes in a signal received at an ultrasonic sensor depending on changes in the wearing state of the wearable device according to an embodiment.
FIG. 12 illustrates a UI provided by the portable device according to an embodiment.
FIG. 13 is a flowchart of a method for measuring body temperature using ultrasonic waves in a wearable device according to an embodiment.
FIG. 14 is a flow diagram of a method for measuring body temperature using ultrasonic waves in a wearable device and a portable device according to an embodiment.

### [Mode for the Invention]

Hereinafter, embodiments of the disclosure will be described in detail with reference to the accompanying drawings so that those skilled in the art can easily carry out the disclosure. However, the disclosure may be implemented in various different forms and is not limited to those embodiments described herein. In the description of the drawings, the same or similar reference symbols may be used for identical or similar components. Additionally, in the drawings and related descriptions, descriptions of well-known functions and configurations may be omitted for clarity and brevity.

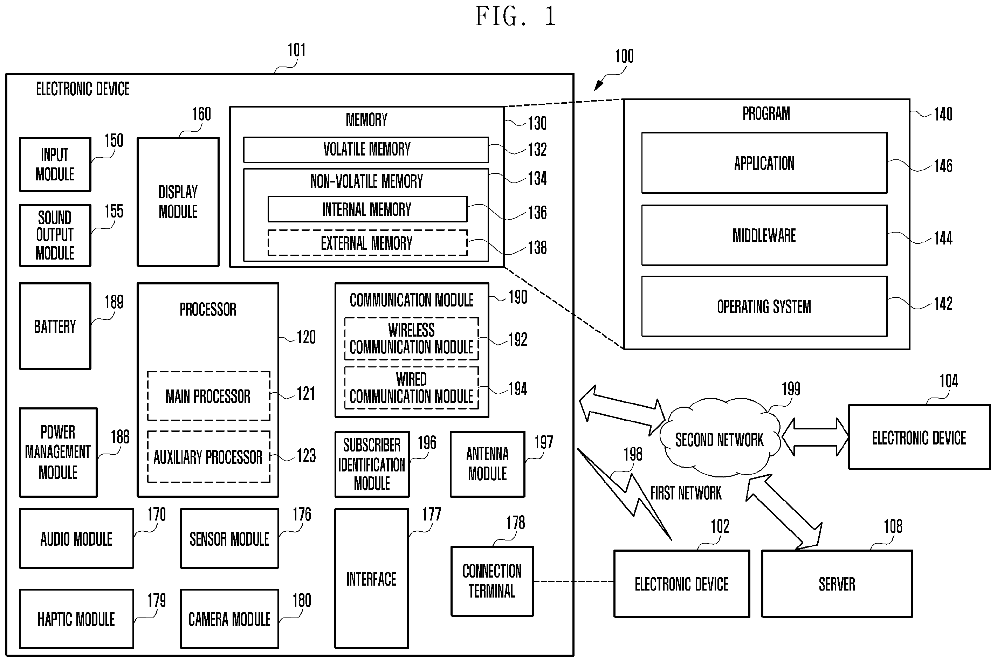

Fig. 1 is a block diagram illustrating an electronic device 101 in a network environment 100 according to various embodiments. With reference to Fig. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thererto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive plural-input and plural-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

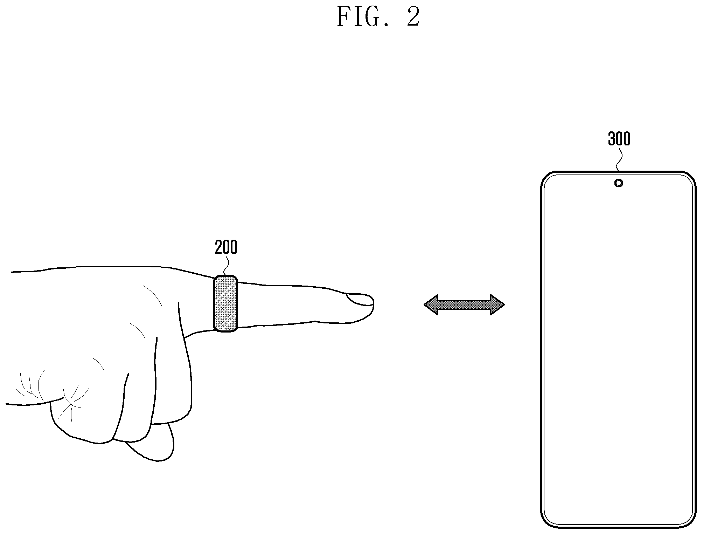

FIG. 2 illustrates a wearable device and a portable device according to an embodiment.

According to an embodiment, the wearable device 200 may be an electronic device that has a biometric information sensing function and a wireless communication function and can be worn on the user's body. With reference to FIG. 2, the wearable device 200 may be a smart ring in the form of a ring worn on a finger of the user, but without being limited thereto, it may be implemented as other types of wearable devices 200 such as a smart watch, a smart band, smart glasses, smart clothing, and smart headphones.

According to an embodiment, the portable device 300 may be an electronic device that can be carried by the user, such as a smartphone or tablet PC. The portable device 300 may provide various user interfaces through various types of input units (e.g., display, microphone, keys, stylus) and output units (e.g., display, speaker, haptic module). The portable device 300 may connect to a network via cellular communication (e.g., 4G LTE, 5G NR) or wireless LAN communication (e.g., Wi-Fi). According to an embodiment, the wearable device 200 and the portable device 300 may communicate with each other through short-range wireless communication. For example, the wearable device 200 and the portable device 300 may be connected through Bluetooth (or Bluetooth low energy) or Wi-Fi (or Wi-Fi direct).

According to an embodiment, the wearable device 200 includes at least one biometric sensor and may measure various biometric information of the user, such as body temperature, heart rate, electrocardiogram, and blood pressure. The wearable device 200 may transmit the measured biometric information to the portable device 300 through established short-range wireless communication.

According to an embodiment, the wearable device 200 may measure the velocity (or travel time) of ultrasonic waves passing through the user's body by using an ultrasonic sensor. The wearable device 200 may include at least one ultrasonic sensor capable of outputting and/or receiving an ultrasonic signal, and when the wearable device 200 is worn on the user's body (e.g., finger), an ultrasonic signal output from one ultrasonic sensor may be detected by the corresponding ultrasonic sensor and/or another ultrasonic sensor located apart therefrom.

According to an embodiment, the wearable device 200 and/or the portable device 300 may measure the user's body temperature based on the velocity (or travel time) of an ultrasonic signal received by at least one ultrasonic sensor. The various embodiments of this document may be categorized into the following embodiments based on the device for measuring body temperature information by analyzing ultrasonic signals and the method for measuring body temperature using ultrasonic signals. According to the first embodiment, the wearable device 200 may set a reference body temperature by using a temperature sensor and analyze ultrasonic signals to determine compensated body temperature information based on the set reference body temperature. According to the second embodiment, the wearable device 200 may determine body temperature information by analyzing ultrasonic signals obtained by plural ultrasonic sensors along various paths (e.g., direct path, reflected path), without using a temperature sensor. According to the third embodiment, the portable device 300 may set the user's reference body temperature and analyze ultrasonic signal information received from the wearable device 200 to determine compensated body temperature information from the reference body temperature. The first to third embodiments described above (and to be described below) are categorized to facilitate understanding of the disclosure, and the various embodiments of this document are not limited thereto. In the following description, descriptions of features that are identical or corresponding to one embodiment may be omitted in some embodiments.

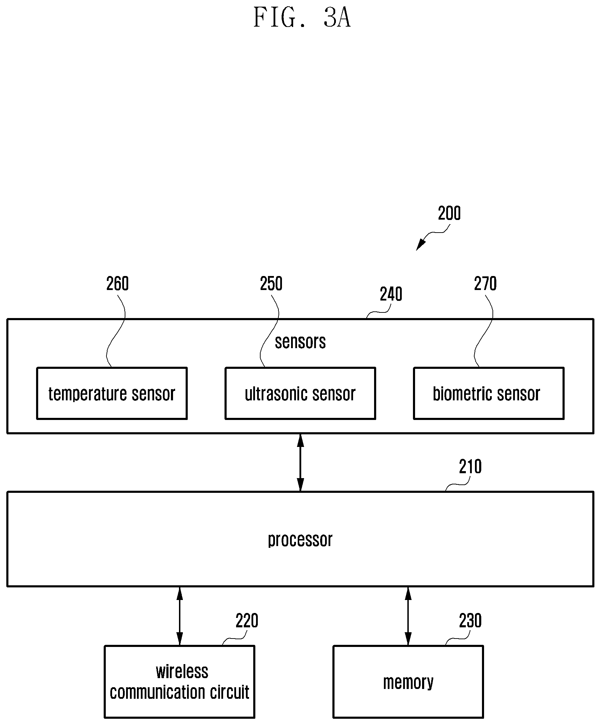

FIG. 3A is a block diagram of a wearable device according to an embodiment.

With reference to FIG. 3A, the wearable device 200 may include various sensors 240, a wireless communication circuit 220, a processor 210, and a memory 230. Various embodiments of this document may be implemented even if some of the illustrated components are omitted or replaced with other components. In addition to the illustrated components, the wearable device 200 may further include at least some of the configurations and/or functions of the electronic device 101 of FIG. 1. 1. At least some of the illustrated components may be electrically, functionally, and/or operably connected to one another.

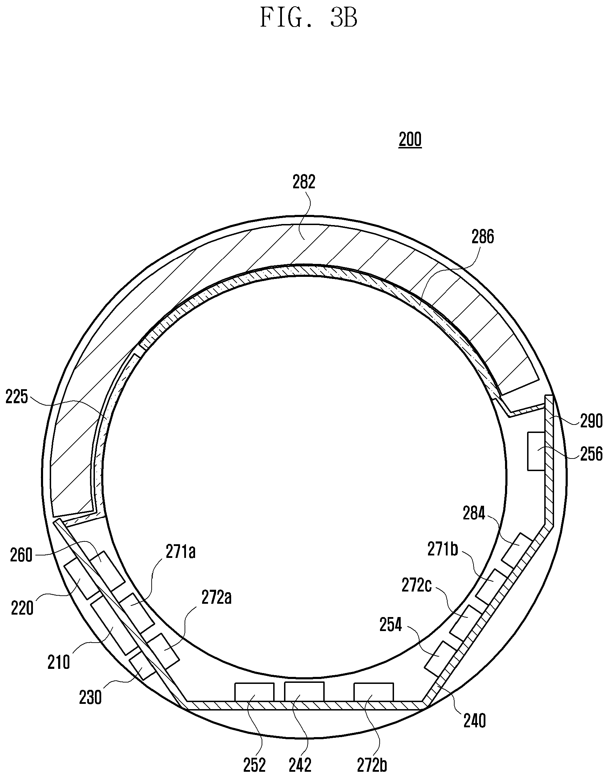

According to an embodiment, the wearable device 200 may include a ring-type housing (not shown). For example, the housing may be formed to enclose an empty central hole, allowing it to be worn on a user's finger. Some of the components of the wearable device 200 may be disposed within the housing, and others may be at least partially exposed to the outside. According to an embodiment, at least one sensor (e.g., biometric sensor 270, temperature sensor 260, and ultrasonic sensor 250) may be disposed toward the central hole in the housing to obtain user's biometric information. The housing structure of the wearable device 200 and the arrangement structure of individual components including sensors will be described in more detail with reference to FIG. 3B.

According to an embodiment, the wearable device 200 may include various types of sensors 240. FIG. 3A illustrates, but not limited to, a temperature sensor 260, an ultrasonic sensor 250, and a biometric sensor 270. The wearable device 200 may include at least some of the configurations and/or functions of the sensor module 176 in FIG. 1.

According to an embodiment, the biometric sensor 270 may obtain various biometric information of the user. For example, the biometric sensor 270 may be, but not limited to, a photoplethysmogram (PPG) sensor that measures pulse waves (plethysmograms) based on optical signals to obtain various biometric information such as heart rate and blood circulation. The wearable device 200 may further include at least one sensor capable of obtaining biometric information such as heart rate and electrocardiogram.

According to an embodiment, the temperature sensor 260 may measure the user's body temperature. For example, the temperature sensor 260 may measure the temperature of the user's skin by utilizing an optical signal (e.g., infrared signal) on the basis of the Stefan-Boltzmann law, which states that radiant energy per unit area is proportional to the fourth power of the absolute temperature. The type of temperature sensor 260 and/or the method of measuring body temperature are not limited thereto, and the temperature sensor 260 defined in this document may include any type of temperature sensor capable of measuring body temperature using information other than ultrasonic waves.

According to an embodiment, the ultrasonic sensor 250 may output and receive ultrasonic signals. For example, the ultrasonic sensor 250 may include at least one piezoelectric element that functions as a transmitter and receiver of ultrasonic signals; when an electrical signal is input to the piezoelectric element, vibration is generated to output ultrasonic waves, and ultrasonic vibration detected by the piezoelectric element is converted into an electrical signal being received as an ultrasonic signal. According to an embodiment, the ultrasonic sensor 250 may include a controller composed of an application-specific integrated circuit (ASIC) and an interface for outputting vibrations to the outside of the housing, and may be connected to the processor 210 and the power management integrated circuit (PMIC) (not shown). Next, various embodiments for measuring the user's body temperature using the ultrasonic sensor 250 will be described, but data sensed by the ultrasonic sensor 250 may be used in various applications other than body temperature measurement.

According to an embodiment, the wearable device 200 may include a plurality of ultrasonic sensors 250 disposed to be spaced apart from each other. For example, the plural ultrasonic sensors 250 may be disposed in a direction toward the central hole in the ring-type housing and be spaced apart from each other by a specific distance to measure changes in the movement velocity of an ultrasonic signal. According to an embodiment, at least some of the plural ultrasonic sensors 250 may be arranged so that ultrasonic signals travel along a direct path that does not pass through the user's bones when the wearable device 200 is worn by the user.

According to an embodiment, the wireless communication circuit 220 may include various hardware and/or software configurations to support wireless communication with external devices. The wireless communication circuit 220 may support short-range wireless communication (e.g., Wi-Fi, Bluetooth), and the wearable device 200 may transmit and receive various data to and from a nearby external device (e.g., portable device 300 in FIG. 2) through the wireless communication circuit 220. The wireless communication circuit 220 may include at least some of the configurations and/or functions of the communication module 190 in FIG. 1.

According to an embodiment, the memory 230 may include a volatile memory and a non-volatile memory so as to temporarily or permanently store various data. The memory 230 may include at least some of the configurations and/or functions of the memory 130 in FIG. 1 and may store the programs 140 in FIG. 1. The memory 230 may store various instructions that can be executed by the processor 210. These instructions may include control commands including arithmetic and logical operations, data movement, and input/output that can be recognized by the processor 210.

According to an embodiment, the processor 210 may be a component capable of performing calculations or data processing related to the control and/or communication of individual components of the wearable device 200, and may be composed of one or more processors. The processor 210 may include at least some of the configurations and/or functions of the processor 120 in FIG. 1. The processor 210 may be operably, functionally, and/or electrically connected to individual components of the wearable device 200, such as the sensors 240, wireless communication circuit 220, and memory 230. Although there are no limitations to the computational and data processing functions that the processor 210 can implement on the wearable device 200, this document will describe various embodiments for measuring (or compensating for) the user's body temperature by using the ultrasonic sensor 250. The operations of the processor 210 to be described later may be executed by loading instructions stored in the memory 230.

In this document, the description that the processor 210 (or, wearable device 200) can perform a specific operation (or, function, job, task) may be interpreted as having substantially the same meaning as that instructions (or, commands, computer program) that cause the wearable device 200 (or, processor 210) to perform the corresponding operation are stored in the memory 230 (e.g., non-volatile memory, storage). Further, the description that the processor 210 can perform a specific operation may be interpreted as having substantially the same meaning as that at least one unspecified processor can perform the corresponding operation.

According to an embodiment, the wearable device 200 may measure the user's body temperature by using the temperature sensor 260. The temperature sensor 260 measures the temperature of the skin surface based on the Stefan-Boltzmann law, so its accuracy may be degraded by external factors. In particular, due to the nature of the wearable device 200 worn on the user's hand or wrist, it is easily affected by the user's clothing or gloves, and because the user's skin is exposed to the external temperature, an error may occur compared with the actual body temperature. Hence, the wearable device 200 may measure the user's current body temperature by using the ultrasonic sensor 250 and/or may compensate for body temperature information measured using the temperature sensor 260.

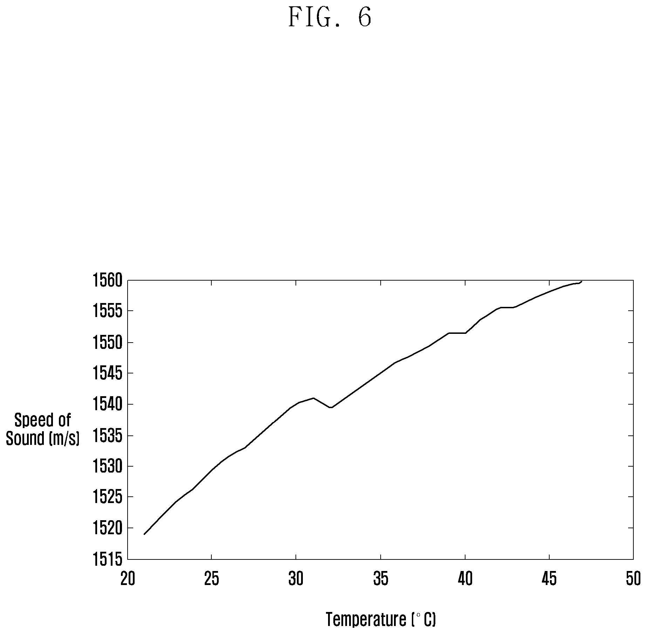

According to an embodiment, the processor 210 may measure the user's current body temperature by using the ultrasonic sensor 250 when worn by the user, or compensate for it based on the reference body temperature. The velocity of an ultrasonic signal may depend on the temperature of the medium. For example, when measured over a medium with the same property (e.g., density), the velocity of the ultrasonic signal may increase substantially in proportion to the temperature of the medium. Since the human body is largely composed of water, the velocity of an ultrasonic wave passing through the body taking into account the temperature of the medium may exhibit a similar trend to the variation in the velocity of the ultrasonic wave due to temperature in water. The relationship between the temperature of the medium and the velocity of the ultrasonic signal will be described in more detail with reference to FIG. 6.

According to an embodiment, since ultrasonic signals are transmitted as vibrations, the ultrasonic signal detected by the ultrasonic sensor 250 may have the form of waves with various amplitudes. According to an embodiment, to determine the arrival time of an ultrasonic signal, the processor 210 may recognize a signal having an amplitude greater than or equal to a first reference value among ultrasonic signals obtained by each ultrasonic sensor 250 as a direct signal, and recognize the first peak point greater than or equal to the first reference value as the arrival time of the direct signal.

According to an embodiment, the wearable device 200 may store, in the memory 230, a mapping table that establishes and stores a mapping between the velocity of an ultrasonic signal and body temperature. For example, the wearable device 200 may establish and store a mapping between body temperature information set via the temperature sensor 260, the ultrasonic sensor 250, or user input through the user interface (UI) and the movement velocity of the ultrasonic signal measured at the corresponding body temperature. Alternatively, the table stored in the memory 230 may be generated by establishing and storing a mapping between the body temperature experimentally determined based on general body characteristics and the movement velocity of the ultrasonic signal, and/or may be generated based on big data including body temperature information and movement velocity collected from various wearable devices 200. When the movement velocity of an ultrasonic signal is measured using the ultrasonic sensor 250, the processor 210 may identify the temperature value mapped to the movement velocity measured by the ultrasonic sensor 250 from the table stored in the memory 230. According to another embodiment, the wearable device 200 may store an equation that can calculate body temperature from the movement velocity of an ultrasonic signal.

According to an embodiment, the processor 210 may correct the table stored in the memory 230 and/or the equation for calculating body temperature based on the user's body information (e.g., body fat percentage, skeletal muscle mass, BMI index) measured using another sensor (e.g., biometric sensor 270). The velocity of an ultrasonic signal may vary depending on the attributes of the medium, and body information such as body fat percentage reflects the attributes of the medium, so the velocity of an ultrasonic signal measured at the same body temperature may also vary when body information changes. Hence, the wearable device 200 may identify the body information measured by the wearable device 200 and/or input on the portable device, and correct the body temperature information mapped to the movement velocity of the ultrasonic signal on the table based on the identified body information.

According to an embodiment, the processor 210 may determine the table and/or equation used to calculate the body temperature based on the wearing state of the wearable device 200. For example, when the user rotates the ring-type wearable device 200, the characteristics of the medium along the path of the ultrasonic signal may change, resulting in a change in the body temperature value corresponding to the movement velocity of the ultrasonic wave. Hence, the wearable device 200 may store a plurality of tables (or equations) corresponding respectively to the wearing states (or angles) of the wearable device 200, and may determine body temperature information corresponding to the movement velocity of the ultrasonic wave by using the table corresponding to the detected wearing state.

According to an embodiment, the wearable device 200 may include a plurality of ultrasonic sensors 250, and may determine user's body temperature information by using ultrasonic signals along various paths via the a plurality of ultrasonic sensors 250. For example, the ultrasonic signal output from a first ultrasonic sensor may be transmitted to a second ultrasonic sensor and a third ultrasonic sensor via a direct path, and may be transmitted to the first, second, and third ultrasonic sensors via reflection paths associated with reflections of the user's bones. Additionally, when the second ultrasonic sensor outputs an ultrasonic signal, and when the second ultrasonic sensor outputs an ultrasonic signal, the ultrasonic signal may be received by each ultrasonic sensor 250 through a plurality of paths. According to an embodiment, the processor 210 may calculate an average (or weighted average) of the movement velocity of ultrasonic waves based on plural pieces of ultrasonic data obtained respectively by the plural ultrasonic sensors 250, and obtain body temperature information corresponding to the calculated average value.

Next, a description will be given of various embodiments for measuring body temperature information by analyzing the movement velocity of an ultrasonic signal.

### [First Embodiment]

According to an embodiment, the wearable device 200 may set a reference body temperature, and determine body temperature information compensated from the set reference body temperature by analyzing ultrasonic signals.

According to an embodiment, the processor 210 may set body temperature information measured by the temperature sensor 260 as the reference body temperature. The temperature sensor 260 may measure the user's body temperature information by utilizing a signal (e.g., infrared signal) different from that of the ultrasonic sensor 250.

According to an embodiment, the processor 210 may receive user's body temperature information via the wireless communication circuit 220 from the portable device. For example, the portable device may provide a user interface (UI) for entering current body temperature on a healthcare application. The user may select the finger on which the wearable device 200 is to be worn through the UI provided on the portable device and directly input the user's body temperature. The processor 210 may set the user's body temperature information received from the portable device as the reference body temperature.

According to an embodiment, the processor 210 may measure the first velocity (or, reference velocity) of ultrasonic waves passing through the user's body by using at least one ultrasonic sensor 250 while the wearable device 200 is worn by the user. For example, the processor 210 may measure the first velocity of ultrasonic waves by using the ultrasonic sensor 250 in response to obtaining body temperature information through the temperature sensor 260 and/or receiving body temperature information from the portable device. That is, the first velocity of ultrasonic waves may be measured at the reference body temperature. The processor 210 may store the reference body temperature and the measured first velocity of ultrasonic waves as reference information in the memory 230. According to an embodiment, when measuring the first velocity (or reference velocity), the processor 210 may determine the first velocity based on the movement velocities obtained respectively from the movement paths of plural ultrasonic signals.

According to an embodiment, after storing the reference information, when a body temperature measurement event occurs, the processor 210 may measure the second velocity of ultrasonic waves by using the ultrasonic sensor 250. For example, the processor 210 may output an ultrasonic signal to measure the second velocity of ultrasonic waves periodically after storing the reference information and/or when the user checks the current body temperature by using a healthcare application on the portable device. According to an embodiment, when measuring the second velocity, the processor 210 may determine the second velocity based on the movement velocities obtained respectively from movement paths of plural ultrasonic signals. Since the user continues to wear the wearable device 200, the medium of the movement paths along which the ultrasonic signals output from each ultrasonic sensor 250 travel may be considered to be the same as when the reference body temperature has been set. Hence, if the current body temperature is higher than the reference body temperature, the ultrasonic signal may travel faster than the reference speed, and if the current body temperature is lower than the reference body temperature, the ultrasonic signal may travel slower than the reference speed. According to an embodiment, to accurately measure the second speed when a body temperature measurement event occurs, the processor 210 may control the ultrasonic signal output of the ultrasonic sensor 250 at a sampling rate higher than or equal to a reference rate.

According to an embodiment, the processor 210 may compensate for the reference body temperature based on the difference between the first velocity (or, reference velocity) and the second velocity of ultrasonic signals and generate compensated body temperature information. For example, the processor 210 may compensate for a value higher than the reference body temperature when the second velocity of the ultrasonic signal is measured to be faster than the first velocity, and may compensate for a value lower than the reference body temperature when the second velocity of the ultrasonic signal is measured to be faster than the first velocity; the larger the difference between the first velocity and the second velocity, the larger the body temperature value for compensation.

According to an embodiment, the processor 210 may transmit the compensated body temperature information via the wireless communication circuit 220 to the portable device. The portable device may provide the received body temperature information to the user through the healthcare application.

According to an embodiment, when the user takes the wearable device 200 off his or her finger and then puts it back on, or when the wearing state (e.g., wearing position, angle) changes, this can be seen as a change in the characteristics of the medium through which the ultrasonic signal travels, so the processor 210 may set the reference body temperature and reference velocity again.

### [Second Embodiment]

According to an embodiment, the wearable device 200 may determine body temperature information by analyzing ultrasonic signals along various paths (e.g., direct path, reflected path) obtained by plural ultrasonic sensors 250, without using the temperature sensor 260.

According to an embodiment, the wearable device 200 may include a plurality of ultrasonic sensors 250, for example, a first ultrasonic sensor, a second ultrasonic sensor, and a third ultrasonic sensor, which are disposed to be spaced apart from each other.

According to an embodiment, the wearable device 200 may store, in the memory 230, a table that establishes and stores a mapping between the velocity of ultrasonic signals and body temperature. For example, the table may be generated by considering the general characteristics of the human body and the physical characteristics of the user of the wearable device 200, and may be generated by experimental and/or statistical calculations as to various users. Alternatively, the table may be a mapping between body temperature and movement velocity by measuring the movement velocity of ultrasonic signals when body temperature is measured through the temperature sensor 260 and/or body temperature information is input by the user. According to another embodiment, the wearable device 200 may store an equation that can calculate the body temperature from the movement velocity of ultrasonic signals.

According to an embodiment, the wearable device 200 may output an ultrasonic signal through the first ultrasonic sensor and calculate the movement velocity of the ultrasonic signal based on the arrival times of the ultrasonic signal received respectively by the first ultrasonic sensor, the second ultrasonic sensor, and the third ultrasonic sensor. Additionally, the wearable device 200 may output an ultrasonic signal through the second ultrasonic sensor and calculate the movement velocity of the ultrasonic signal based on the arrival times of the ultrasonic signal received respectively by the individual ultrasonic sensors 250, and may output an ultrasonic signal through the third ultrasonic sensor and calculate the movement velocity of the ultrasonic signal based on the arrival times of the ultrasonic signal received respectively by the individual ultrasonic sensors 250. A direct signal and a reflected signal may be input to a receiving ultrasonic sensor 250, and the processor 210 may calculate the movement velocity of the ultrasonic signal based on the lengths of the direct path and the reflected path.

According to an embodiment, the processor 210 may calculate the movement time of the ultrasonic signal by analyzing the movement times of the direct signal and the reflected signal obtained from plural ultrasonic signals. For example, the wearable device 200 may calculate the movement velocity of the ultrasonic signal by assigning weights to the first velocity calculated based on the arrival time of the direct signal and the second velocity calculated based on the arrival time of the reflected signal, and calculating the weighted average. According to an embodiment, the processor 210 may obtain body temperature information mapped to the calculated movement velocity from the table stored in the memory 230, and/or may measure the current body temperature information by inputting the movement velocity to the stored equation.

According to an embodiment, the processor 210 may transmit the measured current body temperature information to the portable device via the wireless communication circuit 220. The portable device may provide the user with the received body temperature information through a healthcare application.

### [Third Embodiment]

According to an embodiment, the wearable device 200 may measure the arrival time and/or movement velocity of an ultrasonic signal by using the ultrasonic sensor 250 and transmit the measured data to the portable device. According to an embodiment, the data transmitted from the wearable device 200 to the portable device may include body temperature information measured by the temperature sensor 260 and movement velocities of ultrasonic signals along plural movement paths measured using plural ultrasonic sensors 250.

According to an embodiment, the portable device may set the body temperature information received from the wearable device 200 or the current body temperature input by the user through the UI as the reference body temperature. The portable device may store the first velocity of the ultrasonic wave received from the wearable device 200 as reference information in the memory of the portable device.

According to an embodiment, the portable device may store a table, which establishes and stores a mapping between the velocity of the ultrasonic signal and body temperature, in the memory. Here, the table stored in the memory of the portable device may be the same as the table stored in the memory 230 of the wearable device 200 in the first embodiment.

According to an embodiment, when a body temperature measurement event occurs, the processor 210 may measure the second velocity of the ultrasonic wave by using the ultrasonic sensor 250 and transmit the measured second velocity to the portable device. According to an embodiment, when measuring the second velocity, the processor 210 may determine the second velocity based on the movement velocities obtained respectively along the movement paths of plural ultrasonic signals.

According to an embodiment, the portable device may compensate for the reference body temperature based on the difference between the first velocity (or, reference velocity) and the second velocity of ultrasonic signals and generate compensated body temperature information. For example, the portable device may compensate for a value higher than the reference body temperature when the second velocity of the ultrasonic signal is measured to be faster than the first velocity, and may compensate for a value lower than the reference body temperature when the second velocity of the ultrasonic signal is measured to be faster than the first velocity; the larger the difference between the first velocity and the second velocity, the larger the body temperature value for compensation.

According to an embodiment, the portable device may provide body temperature information received through a healthcare application to the user.

FIG. 3B is a diagram illustrating a sensor arrangement structure of the wearable device according to an embodiment.

FIG. 3B illustrates the sensor arrangement structure of the wearable device implemented as a smart ring. The illustrated arrangement structure is one embodiment, and various embodiments of this document are not limited thereto.

With reference to FIG. 3B, the wearable device 200 may include a ring-type housing (not shown). For example, the housing may be formed to enclose an empty central hole, allowing it to be worn on a user's finger.

According to an embodiment, a plurality of ultrasonic sensors 252, 254 and 256 (e.g., first ultrasonic sensor 252, second ultrasonic sensor 254, third ultrasonic sensor 256) may be arranged on the housing of the wearable device 200. The individual ultrasonic sensors 252, 254 and 256 may be spaced apart from each other, and the plural ultrasonic sensors 252, 254 and 256 may be disposed at the lower end portion of the housing so as to prevent the media (e.g., bones) that reflect ultrasonic signals from being located on the direct paths between the ultrasonic sensors 252, 254 and 256 when the user wears the wearable device 200. Each ultrasonic sensor 252, 254 or 256 may include a controller in the form of an application-specific integrated circuit (ASIC) and an interface for outputting vibrations to the outside of the housing, and may be connected to the processor 210 and the power management integrated circuit (PMIC) 284.

According to an embodiment, the wearable device 200 may include, as an example of a biometric sensor, a photoplethysmogram (PPG) sensor. The PPG sensor may include a plurality of emitters 272a, 272b and 272c that output optical signals, and a plurality of receivers 271a and 271b that receive optical signals output from the emitters 272a, 272b and 272c. The emitters 272a, 272b and 272c of the PPG sensor may be arranged to be spaced apart from the receivers 271a and 271b thereof.

According to an embodiment, the wearable device 200 may include a temperature sensor 260 that measures the temperature of the user's skin by using an optical signal (e.g., infrared signal). The temperature sensor 260 may be positioned next to the first receiver 271a of the PPG sensor, but its location is not limited thereto.

According to an embodiment, the individual sensors may be mounted on a flexible printed circuit board (FPCB) 290. With reference to FIG. 3B, the FPCB 290 may include a bent portion for placement on the ring-shaped housing. According to another embodiment, a plurality of FPCBs 290 electrically connected to each other may be arranged within the housing, or an FPCB 290 formed as an overall curved surface along the curved surface of the housing may be arranged.

According to an embodiment, the processor 210, the wireless communication circuit 220, and the memory 230 may be mounted on the surface of the FPCB 290 opposite to the surface where the sensors are arranged. The placement of the processor 210, the wireless communication circuit 220, and the memory 230 is not limited thereto.

According to an embodiment, a battery 282 may be placed on the opposite side of the location where the sensors are disposed. The battery 282 may be connected to a charging interface 286, and the charging interface 286 may be electrically connected to, via the FPCB 290, the PMIC 284 mounted on the FPCB 290. The PMIC may manage the power delivered from the battery 282 to individual components of the wearable device 200.

According to an embodiment, an antenna 225 is connected to the FPCB 290, and the wearable device 200 may transmit and receive various data to and from an external device (e.g., portable device 300 in FIG. 2) through the antenna 225.

The arrangement structure of FIG. 3B corresponds to an embodiment, and various embodiments of this document are not limited thereto.

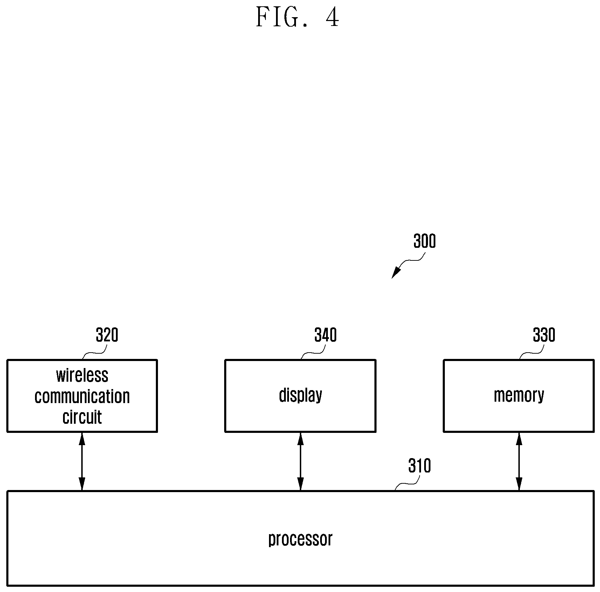

FIG. 4 is a block diagram of a portable device according to an embodiment.

With reference to FIG. 4, the portable device 300 may include a display 340, a wireless communication circuit 320, a memory 330, and a processor 310. Various embodiments of this document can be implemented even if some of the illustrated components are omitted or replaced with other components. In addition to the illustrated components, the portable device 300 may further include at least some of the configurations and/or functions of the electronic device 101 in FIG. 1. At least some of the depicted components may be electrically, functionally, and/or operably connected to one another.

According to an embodiment, the display 340 may display various images provided by the processor 310. For example, the display 340 may be implemented with, but not limited to, any one of liquid crystal display (LCD), light-emitting diode (LED) display, organic light-emitting diode (OLED) display, micro electromechanical systems (MEMS) display, or electronic paper display. The display 340 may be composed of a touch screen that detects touch input and/or proximity touch (or hovering) input using a user's body part (e.g., finger) or an input unit (e.g., stylus pen). The display 340 may include at least some of the configurations and/or functions of the display module 160 in FIG. 1. According to an embodiment, the display 340 may be a flexible display having at least a portion that is flexible, and the portable device 300 may be implemented in various form factors (e.g., foldable device, slidable device) in which the display area of the flexible display can be changed.

According to an embodiment, the wireless communication circuit 320 may include various hardware and/or software configurations to support wireless communication with an external device. The wireless communication circuit 320 may support short-range wireless communication (e.g., Wi-Fi, Bluetooth) and cellular wireless communication (e.g., 4G LTE, 5G NR). The communication module may include at least some of the configurations and/or functions of the communication module 190 in FIG. 1.

According to an embodiment, the memory 330 may include a volatile memory and a non-volatile memory so as to temporarily or permanently store various data. The memory 330 may include at least some of the configurations and/or functions of the memory 130 in FIG. 1 and may store the programs 140 in FIG. 1. The memory 330 may store various instructions that can be executed by the processor 310. These instructions may include control commands including arithmetic and logical operations, data movement, and input/output that can be recognized by the processor 310.

According to an embodiment, the processor 310 may be a component capable of performing calculations or data processing related to the control and/or communication of individual components of the portable device 300, and may be composed of one or more processors. The processor 310 may include at least some of the configurations and/or functions of the processor 120 in FIG. 1. The processor 310 may be operably, functionally, and/or electrically connected to individual components of the wearable device 300, such as the display 340, wireless communication circuit 320, and memory 330. Although there are no limitations to the computational and data processing functions that the processor 310 can implement on the portable device 300, this document describes various embodiments for receiving data measured by an ultrasonic sensor from the wearable device (e.g., wearable device 200 in FIGS. 2, 3A and 3B) connected via short-range wireless communication, and measuring (or compensating for) the user's body temperature based on the received data. The operations of the processor 310 to be described later may be executed by loading instructions stored in the memory 330.

In this document, the description that the processor 310 (or, portable device 300) can perform a specific operation (or, function, job, task) may be interpreted as having substantially the same meaning as that instructions (or, commands, computer program) that cause the portable device 300 (or, processor 310) to perform the corresponding operation are stored in the memory 330 (e.g., non-volatile memory, storage). Further, the description that the processor 310 can perform a specific operation may be interpreted as having substantially the same meaning as that at least one unspecified processor can perform the corresponding operation.

According to an embodiment, the processor 310 may execute a healthcare application stored in the memory 330. This healthcare application may register the initial body temperature when the user wears the wearable device, provide body temperature information measured by the wearable device or portable device 300, and provide a user interface (UI), which provides various biometric information (e.g., heart rate, electrocardiogram, blood pressure) measured by the wearable device and additional information related to the biometric information, through the display 340.

Next, a description will be given of operations of the portable device 300 in various embodiments that measure body temperature information by analyzing the movement velocity of ultrasonic signals. The first embodiment, second embodiment, and third embodiment to be described below may correspond to the first embodiment, second embodiment, and third embodiment described above in relation to FIG. 3A.

### [First Embodiment]

According to an embodiment, when the healthcare application is executed, the processor 310 may provide a UI for setting the reference body temperature via the display 340. For example, the UI may include various screens that allow the user to enter the hand (e.g., left or right) on which the wearable device will be worn, the finger (e.g., thumb, index finger, middle finger, ring finger, little finger), and the current body temperature. The processor 310 may input body temperature information based on the user's input while the UI is displayed. The processor 310 may transmit the input body temperature information via the wireless communication circuit 320 to the wearable device.

According to an embodiment, the wearable device may measure the first velocity of the ultrasonic signal at the reference body temperature and store it as reference information. After storing the reference information, when a body temperature measurement event occurs, the wearable device may measure the second velocity of ultrasonic waves by using the ultrasonic sensor. The wearable device may compensate for the reference body temperature based on the difference between the first velocity (or, reference velocity) and the second velocity of ultrasonic signals to generate compensated body temperature information. The wearable device may transmit the compensated body temperature information to the portable device 300.

According to an embodiment, the processor 310 may provide the body temperature information received from the wearable device to the user via the healthcare application.

### [Second Embodiment]

According to an embodiment, the wearable device may determine body temperature information by analyzing ultrasonic signals along various paths (e.g., direct path, reflected path) received by a plurality of ultrasonic sensors.

According to an embodiment, the portable device 300 may receive measured body temperature information from the wearable device, and the processor 310 may provide the received body temperature information to the user through the healthcare application.

### [Third Embodiment]

According to an embodiment, when a healthcare application is executed, the processor 310 may provide a UI for setting a reference body temperature via the display 340. For example, the UI may include various screens that allow the user to enter the hand (e.g., left, right) on which the wearable device will be worn, the finger (e.g., thumb, index finger, middle finger, ring finger, little finger), and the current body temperature. The processor 310 may input body temperature information based on the user's input while the UI is displayed.

According to an embodiment, the portable device 300 may receive body temperature information measured by the temperature sensor of the wearable device via the wireless communication circuit 320. The processor 310 may set the body temperature information input through the UI or the body temperature information received from the wearable device as the reference body temperature and store it in the memory 330.

According to an embodiment, the wearable device being worn by the user may measure the first velocity (or, reference velocity) of ultrasonic waves passing through the user's body by using at least one ultrasonic sensor. The portable device 300 may receive measured first speed data from the wearable device via the wireless communication circuit 320.

According to an embodiment, the processor 310 may store the reference body temperature and the first velocity of ultrasonic waves received from the wearable device as reference information in the memory 330.

According to an embodiment, when a body temperature measurement event occurs, the processor 310 may request the wearable device to measure the movement velocity of ultrasonic signals. The wearable device may measure the second velocity of ultrasonic waves by using an ultrasonic sensor and transmit the measured second velocity to the portable device 300. According to an embodiment, the second velocity may include movement velocities obtained respectively from the movement paths of plural ultrasonic signals.

According to an embodiment, the processor 310 may compensate for the reference body temperature based on the difference between the first velocity (or, reference velocity) and the second velocity of ultrasonic signals and generate compensated body temperature information. For example, the portable device may compensate for a value higher than the reference body temperature when the second velocity of the ultrasonic signal is measured to be faster than the first velocity, and may compensate for a value lower than the reference body temperature when the second velocity of the ultrasonic signal is measured to be faster than the first velocity; the larger the difference between the first velocity and the second velocity, the larger the body temperature value for compensation.

According to an embodiment, the processor 310 may provide the compensated body temperature information through the UI of the healthcare application.

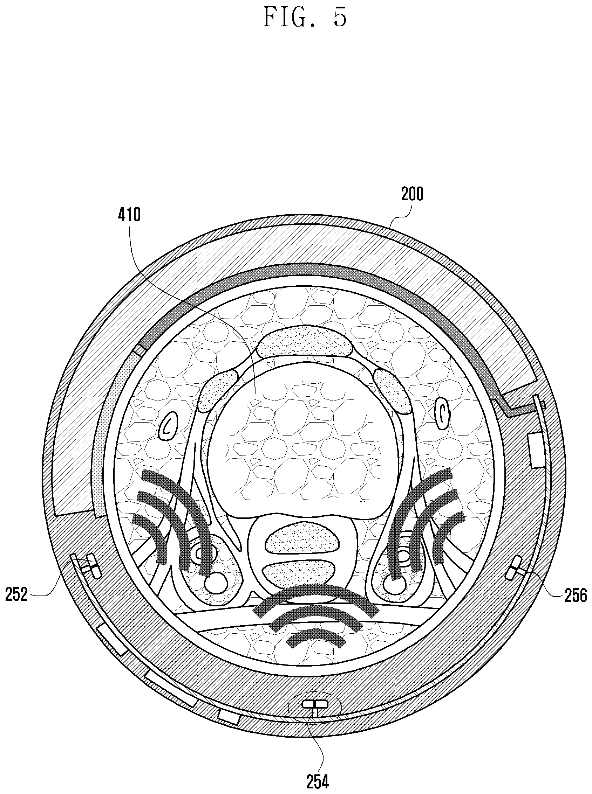

FIG. 5 illustrates the principle of the ultrasonic sensor in the wearable device according to an embodiment.

According to an embodiment, the wearable device 200 may include at least one ultrasonic sensor (e.g., ultrasonic sensor 250 in FIG. 3A or 3B) disposed on the side of the hole in the ring-type housing. With reference to FIG. 5, the wearable device 200 may include a first ultrasonic sensor 252, a second ultrasonic sensor 254, and a third ultrasonic sensor 256, which are disposed to be spaced apart from each other, and the number and/or arrangement positions of ultrasonic sensors are not limited thereto.

According to an embodiment, each of the ultrasonic sensors 252, 254 and 256 may include at least one piezoelectric element that functions as a transmitter and receiver for ultrasonic signals. When an electrical signal is input to the piezoelectric element, vibrations are generated to thereby output ultrasonic waves. When an ultrasonic vibration is detected by the piezoelectric element, it is converted into an electrical signal to thereby receive an ultrasonic signal. Each ultrasonic sensor 252, 254 or 256 may output an ultrasonic signal of a specified frequency (e.g., about 10 MHz).

According to an embodiment, when an ultrasonic signal is output from one ultrasonic sensor, the ultrasonic signal may be received by that ultrasonic sensor and/or another ultrasonic sensor. For example, an ultrasonic signal output from the first ultrasonic sensor 252 may pass through and/or be reflected by body tissue being media, and be received by at least one of the first ultrasonic sensor 252, the second ultrasonic sensor 254, or the third ultrasonic sensor 256. Human body tissues may be composed of various media, and ultrasonic signals may pass through some media while they cannot pass through some other media but be reflected. For example, ultrasonic signals may pass through epithelial tissue, muscle tissue, and nervous tissue, and be reflected by the bone 410. In the following description, soft tissues are defined as media that allow ultrasonic signals to pass through without reflection, such as epithelial, muscle, and nervous tissues. The ultrasonic signal output from the first ultrasonic sensor 252 may pass through soft tissue and be received by the second ultrasonic sensor 254 and/or the third ultrasonic sensor 256, and may be reflected by the bone 410 and be received by the first ultrasonic sensor 252, the second ultrasonic sensor 254 and/or the third ultrasonic sensor 256.

FIG. 6 is a graph showing the relationship between the temperature of a medium and the velocity of ultrasonic waves according to an embodiment.

According to an embodiment, the wearable device (e.g., wearable device 200 in FIGS. 3A and 3B) (or, portable device (e.g., portable device 300 in FIG. 4)) may measure the velocity of an ultrasonic signal according to a pulse-echo scheme and measure (or, compensate for) the user's body temperature based on the measured velocity. The velocity of an ultrasonic signal may vary depending on the attributes of the medium through which it passes. In FIG. 6, the x-axis represents the temperature of the medium (e.g., soft tissue) through which the ultrasonic signal passes (or, user's body temperature), and the y-axis represents the velocity of the ultrasonic signal (m/s).

FIG. 6 is a graph showing the temperature of the medium and the velocity of an ultrasonic signal measured through a tissue phantom. With reference to FIG. 6, when measured on a medium of the same attributes, it may be identified that the speed of the ultrasonic signal increases substantially proportionally as the temperature of the medium increases. Since most of the human body is made up of water, the velocity of the ultrasonic wave passing through the body depending on the temperature of the medium may have a similar tendency to the change in velocity of the ultrasonic wave depending on the temperature in water.

With reference to the graph in FIG. 6, when an ultrasonic signal passes through a medium with the same attributes (e.g., finger), the higher the temperature of the medium, the faster the ultrasonic signal may travel. Hence, if the physical properties (e.g., density) of the user's body being a medium do not change, the movement velocity may be calculated from the travel time between the output and reception of the ultrasonic signal received by each ultrasonic sensor, and the user's body temperature may be measured from this.

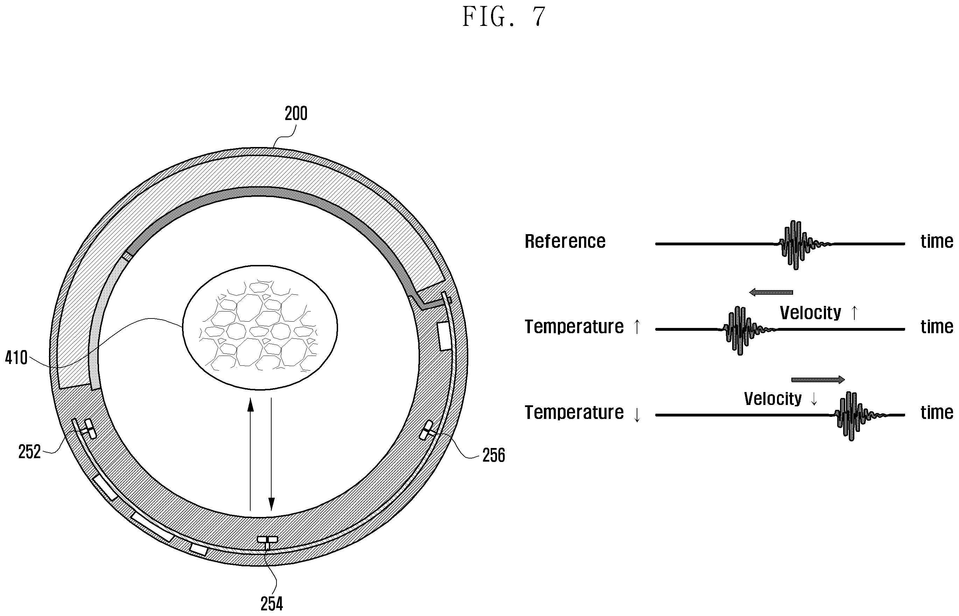

FIG. 7 is a diagram for explaining changes in the velocity of an ultrasonic signal with changes in body temperature according to an embodiment.

With reference to FIG. 7, the wearable device 200 may include a first ultrasonic sensor 252, a second ultrasonic sensor 254, and a third ultrasonic sensor 256, which are disposed in the direction toward the hole in the ring-type housing.

According to an embodiment, the wearable device 200 (or, portable device) may set a reference body temperature, measure the velocity of an ultrasonic signal passing through a body part (e.g., finger) by using the ultrasonic sensors 252, 254 and 256 in a state where the reference body temperature is set, and store the reference body temperature and the measured reference velocity of the ultrasonic signal as reference information in the memory (e.g., memory 230 in FIG. 3A, memory 330 in FIG. 4). For example, the wearable device 200 may measure the body temperature by using a temperature sensor (e.g., temperature sensor 260 in FIG. 3A) other than the ultrasonic sensor 252, 254 or 256 in a non-ultrasonic manner (e.g., infrared waves), and/or may set a body temperature directly input by the user on the user interface of the portable device as the reference body temperature. The velocity of an ultrasonic wave passing through the human body may vary depending on the body temperature, and the measured reference velocity of the ultrasonic signal may be the velocity of the ultrasonic signal at the reference body temperature.

According to an embodiment, after storing the reference information, the wearable device 200 (or, portable device) may measure the velocity of an ultrasonic signal by using at least one ultrasonic sensor, and check whether the body temperature has changed based on the difference between the measured velocity and the reference velocity of the reference information. With reference to FIG. 7, if the body temperature has increased compared to the time when the reference information is set, the ultrasonic signal may be detected relatively earlier by the receiving ultrasonic sensor, indicating a faster travel velocity of the ultrasonic signal. Further, if the body temperature has decreased compared to the time when the reference information is set, the ultrasonic signal may be detected relatively later by the receiving ultrasonic sensor, indicating a slower travel velocity of the ultrasonic signal.

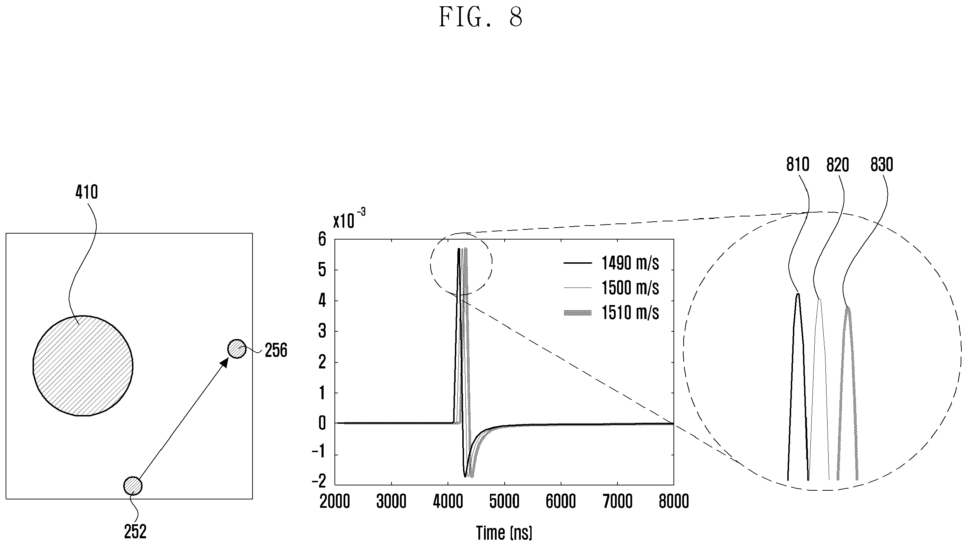

FIG. 8 illustrates a signal detected at the receiving end of an ultrasonic sensor according to an embodiment.

In FIG. 8, the x-axis may represent the time elapsed after the transmitting end outputs an ultrasonic signal, and the y-axis may represent the intensity of the ultrasonic signal detected at the receiving end. According to an embodiment, when an ultrasonic signal is received at the receiving end of an ultrasonic sensor, the intensity of the ultrasonic signal may be detected through the intensity of pressure and converted into an electrical signal. According to an embodiment, the wearable device 200 may include plural ultrasonic sensors (e.g., first ultrasonic sensor 252, second ultrasonic sensor 254, and third ultrasonic sensor 256 in FIG. 5), which are disposed to be spaced apart from each other. An ultrasonic signal output from one ultrasonic sensor may pass through body tissues and be delivered to another ultrasonic sensor, or be reflected by the bone 410 and delivered to that ultrasonic sensor or another ultrasonic sensor. FIG. 8 shows the arrival time of an ultrasonic signal according to the body temperature, where the ultrasonic signal output from the first ultrasonic sensor 252 passes through soft tissues of the body along a direct path and arrives at the third ultrasonic sensor 256.

With reference to FIG. 8, the bone 410 that reflects the ultrasonic signal may be not on the direct path between the first ultrasonic sensor 252 and the third ultrasonic sensor 256, and only soft tissues that transmit the ultrasonic signal may be present thereon. Since the distance between the first ultrasonic sensor 252 and the third ultrasonic sensor 256 is fixed, the movement velocity of the ultrasonic signal output from the first ultrasonic sensor 252 and received by the third ultrasonic sensor 256 may be determined according to the body temperature of the user corresponding to the soft tissue.

The graph of FIG. 8 illustrates ultrasonic signals received by the third ultrasonic sensor 256 measured at three temperatures when the first ultrasonic sensor 252 outputs a 10 MHz, 1 cycle ultrasonic signal while keeping other conditions fixed. With reference to the graph, when the body temperature is T2 (e.g., about 36.5 degrees), the velocity of the ultrasonic signal was measured to be about 1500 m/s; when the body temperature is T1 (e.g., about 37 degrees) which is higher than T2, the velocity of the ultrasonic signal was measured to be 1510 m/s; when the body temperature is T3 (e.g., about 36 degrees) which is lower than T2, the velocity of the ultrasonic signal was measured to be 1490 m/s.

According to an embodiment, for a wave-shaped ultrasonic signal, the wearable device 200 may determine the first peak point 810, 820 or 830 with an amplitude greater than or equal to a reference value to be the arrival time of the ultrasonic signal. With reference to FIG. 8, for the ultrasonic signals measured at different temperatures, the first peak point 810, 820 or 830 may be detected earlier as the body temperature is higher.

According to an embodiment, the wearable device 200 in the form of a smart ring must be small enough to be worn on the user's finger. Consequently, the distance between ultrasonic sensors cannot be large, and the length of the direct path of the ultrasonic signal must be short accordingly. Further, the human body's temperature fluctuates within a limited range, typically around 35 to 40 degrees Celsius. Hence, the arrival time of the direct signal at the receiving ultrasonic sensor can only show small changes of about tens to hundreds of ns (nanoseconds) even if the body temperature changes.

According to an embodiment, to more accurately detect subtle changes in the movement velocity of an ultrasonic signal due to body temperature changes, the wearable device 200 may measure ultrasonic signals at a high sampling rate during body temperature measurement. For example, for more accurate body temperature measurement, the wearable device 200 may increase the sampling rate for outputting and detecting the ultrasonic signal in the case where the reference velocity of the ultrasonic signal is measured for setting the reference information, the body temperature (or, movement velocity of the ultrasonic signal) is measured to have changed significantly by more than the threshold value compared to the reference information, or the user checks the body temperature of the user through the wearable device 200 or the portable device.

According to an embodiment, to more accurately detect subtle changes in the movement velocity of ultrasonic signals due to body temperature changes, the wearable device 200 may measure movement velocities of ultrasonic signals along plural direct paths (e.g., first ultrasonic sensor 252 - second ultrasonic sensor, first ultrasonic sensor 252 - third ultrasonic sensor 256) and use the plural pieces of measurement data to measure a body temperature change. In addition, the wearable device 200 may measure a body temperature change by further utilizing a reflection signal on a reflected path, where the reflection signal is output from the transmitting ultrasonic sensor, reflected by the bone 410, and received by the receiving ultrasonic sensor.

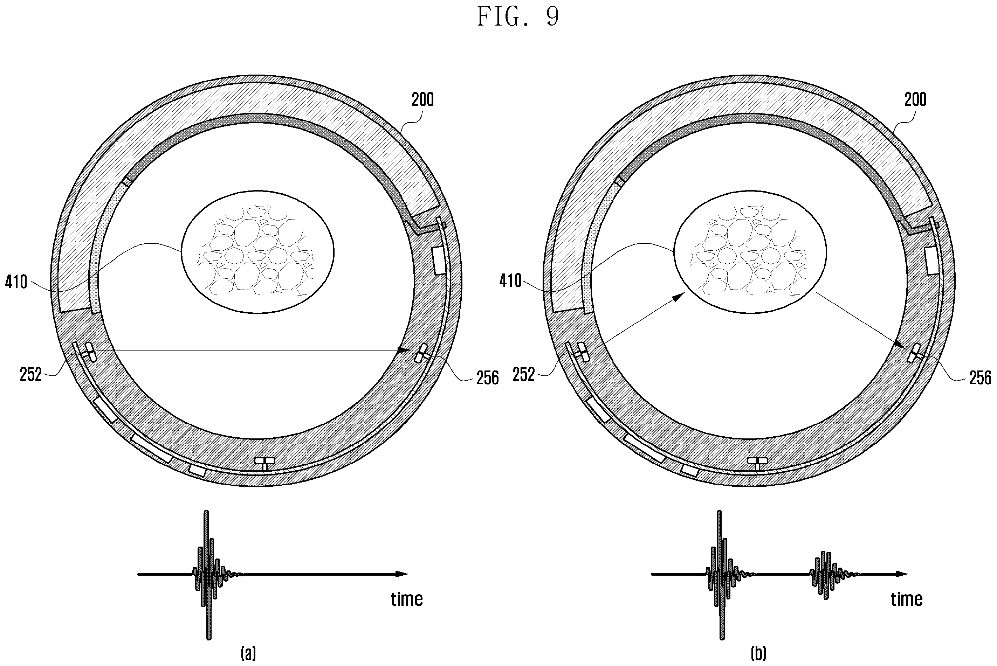

FIG. 9 illustrates signals measured on the direct path and reflected path of ultrasonic signals according to an embodiment.

According to an embodiment, the wearable device 200 may include plural ultrasonic sensors (e.g., first ultrasonic sensor 252, second ultrasonic sensor 254, and third ultrasonic sensor 256 in FIG. 5) arranged to be spaced apart from each other. The ultrasonic signal output from one ultrasonic sensor may pass through body tissues and be transmitted to another ultrasonic sensor, or be reflected by the bone 410 and transmitted to that ultrasonic sensor or another ultrasonic sensor. FIG. 9 illustrates a direct signal that is output from the first ultrasonic sensor 252, passes through the soft tissue and received by the third ultrasonic signal, and a reflection signal that is reflected by the bone 410 and received by the third ultrasonic signal.

With reference to part (a) of FIG. 9, the direct signal may be an ultrasonic signal transmitted along a direct path from the first ultrasonic sensor 252 through the soft tissue of the body to the third ultrasonic sensor 256. With reference to part (b) of FIG. 9, the reflection signal may be an ultrasonic signal, which is output from the first ultrasonic sensor 252, reflected by the bone (410), and transmitted to the third ultrasonic sensor 256, along a reflected path.

Since the direct path is shorter than the reflected path, the direct signal of the ultrasonic signal output from the first ultrasonic sensor 252 may arrive at the third ultrasonic sensor 256 at time t₁, and the reflection signal may arrive at the third ultrasonic sensor 256 at a later time t₂. Additionally, since only a portion of the ultrasonic signal is reflected by the bone 410 and transmitted to the third ultrasonic sensor 256 as a reflection signal, the intensity of the reflection signal may be less than that of the direct signal. According to an embodiment, the wearable device 200 may determine the arrival time of the direct signal to be the point in time (or, first peak point) at which an ultrasonic signal with an intensity higher than or equal to a threshold value is first detected by the third ultrasonic sensor 256 after the first ultrasonic sensor 252 outputs the ultrasonic signal. In addition, the wearable device 200 may determine the arrival time of the reflection signal to be the point in time at which an ultrasonic signal with an intensity lower than the threshold value within a preset range is first detected after the third ultrasonic sensor 256 detects the direct signal.

Considering the structural characteristics of the ring-type wearable device 200, the length of the direct path may always be the same because the positions of the ultrasonic sensors 252, 254 and 256 are fixed. Hence, in the case of a direct signal, the distance between the first ultrasonic sensor 252 and the third ultrasonic sensor 256 may be divided by the travel time, which is the difference between the time the first ultrasonic sensor 252 outputs an ultrasonic signal and the time the third ultrasonic sensor 256 receives the ultrasonic signal, and the corresponding result of division may be calculated to be the movement velocity of the direct signal. Since the movement velocity of the ultrasonic signal may be determined according to the temperature of the medium, the wearable device 200 (or, portable device) may determine the temperature value corresponding to the calculated movement velocity of the direct signal to be the current body temperature.

Since the direct path corresponds to the straight-line distance between ultrasonic sensors, the length of the direct path may remain constant even when the wearing state of the wearable device 200 is changed. However, the direct path has the disadvantage of not being able to accurately measure the body temperature even with slight errors due to its short length. For more precise analysis of the arrival time (or, movement velocity) of the ultrasonic signal, a longer travel distance is advantageous, and analyzing various paths may improve accuracy. As the ultrasonic wave travels longer, the arrival time at the receiving ultrasonic sensor may be delayed at the same velocity. This may result in a greater difference in arrival times for the same velocity change compared to signals that travel a shorter distance and arrive quickly. Therefore, measuring ultrasonic signals that have traveled as far as possible may be more advantageous for measuring precise temperature changes.

According to an embodiment, the wearable device 200 (or, portable device) may measure the travel time of the ultrasonic signal and the current body temperature (or, a change from the reference body temperature) by analyzing the travel time of the direct signal and the travel time of the reflection signal. For example, the wearable device 200 may calculate the movement velocity of the ultrasonic signal by assigning weights to the first velocity calculated based on the arrival time of the direct signal and the second velocity calculated based on the arrival time of the reflection signal, and calculating the weighted average. In addition, the wearable device 200 may output ultrasonic signals from plural ultrasonic sensors and measure the user's body temperature by analyzing the multi-path ultrasonic signals received by the individual ultrasonic sensors.

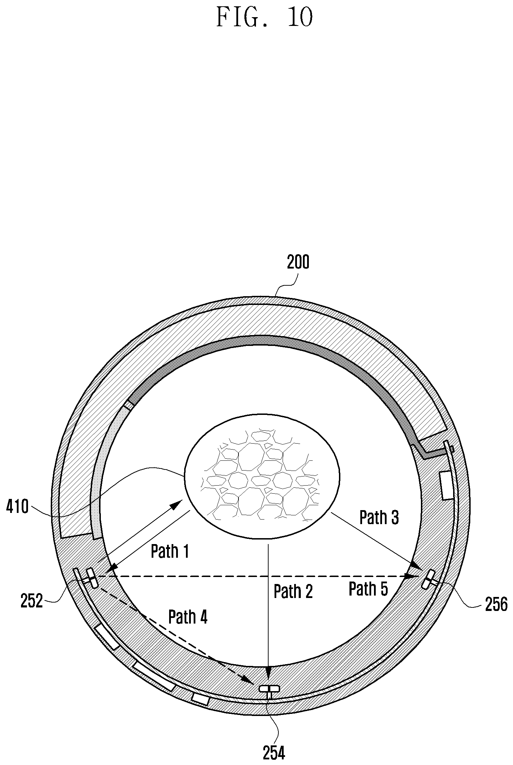

FIG. 10 illustrates transmission paths of ultrasonic signals according to an embodiment.

According to an embodiment, the wearable device 200 may include a plurality of ultrasonic sensors, and to measure the user's body temperature, it may output ultrasonic signals from plural ultrasonic sensors and receive ultrasonic signals at plural ultrasonic sensors, and analyze the travel time of each received ultrasonic signal to measure the user's current body temperature (or, change from the reference body temperature).

FIG. 10 illustrates the paths of an ultrasonic signal, which is output from the first ultrasonic sensor 252 and transmitted to the first ultrasonic sensor 252, the second ultrasonic sensor 254, and the third ultrasonic sensor 256.

With reference to FIG. 10, the ultrasonic signal output from the first ultrasonic sensor 252 may be transmitted to the second ultrasonic sensor 254 and the third ultrasonic sensor 256 respectively through direct paths path 4 and path 5. In addition, the ultrasonic signal output from the first ultrasonic sensor 252 may be reflected by the bone 410 and transmitted to the first ultrasonic sensor 252, the second ultrasonic sensor 254, and the third ultrasonic sensor 256 respectively through reflected paths path 1, path 2, and path 3.

According to an embodiment, the wearable device 200 may sequentially output an ultrasonic signal from each of the ultrasonic sensors and calculate the travel time of the ultrasonic signal by using the arrival times of the ultrasonic signals received through plural paths by the individual ultrasonic sensors. For example, as shown in FIG. 10, the first ultrasonic sensor 252 may output an ultrasonic signal and every ultrasonic sensor may receive the ultrasonic signal to calculate the movement velocity; the second ultrasonic sensor 254 may output an ultrasonic signal and every ultrasonic sensor may receive the ultrasonic signal to calculate the movement velocity; and the third ultrasonic sensor 256 may output an ultrasonic signal and every ultrasonic sensor may receive the ultrasonic signal to calculate the movement velocity. When outputting an ultrasonic signal from one ultrasonic sensor, it is possible to obtain movement velocities corresponding to five paths (e.g., paths 1 to 5 in FIG. 10). Hence, by outputting ultrasonic signals from three ultrasonic sensors, the data obtained may include movement velocities corresponding to a total of 15 paths. As described above, the greater the number of data values acquired, the more precise the system can be for measuring the body temperature.

According to an embodiment, the wearable device 200 may store and process the arrival times of ultrasonic signals obtained respectively by individual ultrasonic sensors, or may calculate the ratio of travel distances (or, the ratio of arrival times) of individual ultrasonic signals based on the arrival times (or travel velocities) at the receiving ultrasonic sensors and use this for temperature measurement. For example, the wearable device 200 may predict the movement path of an ultrasonic signal based on the locations of the transmitting and receiving sensors, and designate each movement path as a single path. In this case, the length of each movement path (or, travel distance of an ultrasonic signal) may be constant, independent of the movement velocity. Since the travel distance of each movement path (e.g., paths 1 to 5) is constant, the ultrasonic signals received by each ultrasonic sensor are measured at the same body temperature. Since the temperature of the medium is the same, the movement velocity of the ultrasonic signal along each movement path at a specific time may be substantially the same. As a result, the wearable device 200 may obtain signals transmitted through various paths by using plural ultrasonic sensors, and measure the body temperature based on the arrival time of each ultrasonic signal and the ratio of the arrival times.

According to an embodiment, the wearable device 200 may calculate an average (or weighted average) of the movement velocities of ultrasonic signals measured along each movement path and calculate the current body temperature corresponding to the average of the calculated movement speeds velocities. According to another embodiment, the wearable device 200 may calculate an average (or weighted average) of the movement velocities of ultrasonic signals measured along each movement path, calculate a difference between the average of the calculated movement velocities and the movement velocity of the ultrasonic signal in the reference information, and compensate for the reference body temperature based on the calculated difference to thereby calculate compensated body temperature information. According to an embodiment, the wearable device 200 may assign a higher weight to the direct path when calculating the weighted average of movement velocities on each movement path.

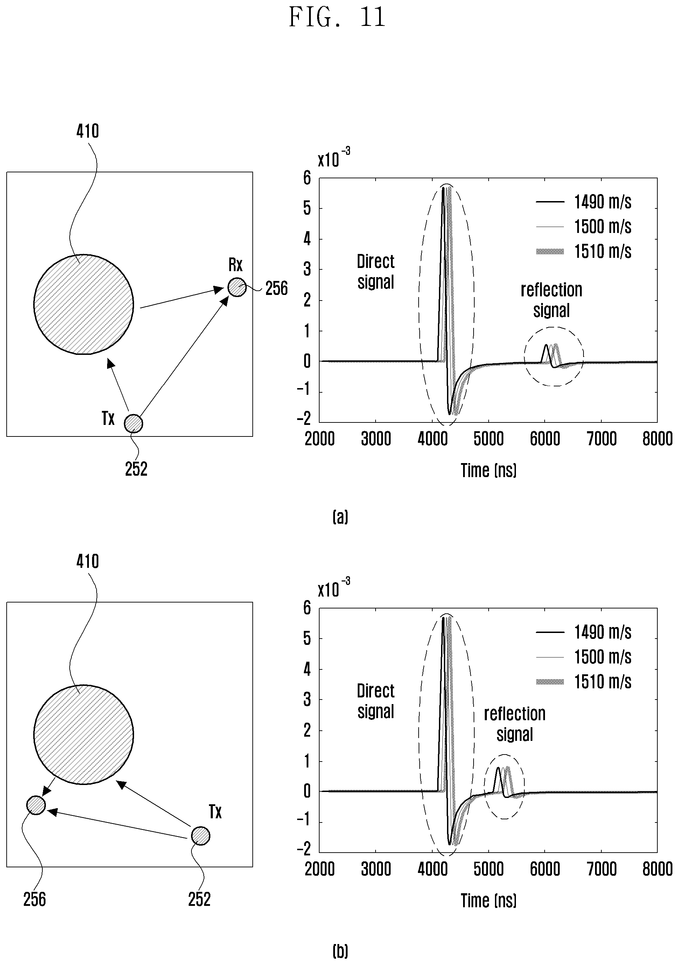

FIG. 11 illustrates changes in a signal received at an ultrasonic sensor depending on changes in the wearing state of the wearable device according to an embodiment.

According to an embodiment, the wearable device 200 includes plural ultrasonic sensors, and the wearable device 200 (or, portable device) may determine user's body temperature information by using ultrasonic signals along various paths generated via the plural ultrasonic sensors 252, 254 and 256. For example, the ultrasonic signal output from the first ultrasonic sensor 252 may be transmitted to the second ultrasonic sensor 254 and the third ultrasonic sensor 256 through direct paths, or may be reflected by the user's bone 410 and transmitted to the first ultrasonic sensor 252, the second ultrasonic sensor 254, and the third ultrasonic sensor 256 through reflected paths. In addition, when an ultrasonic signal is output from the second ultrasonic sensor 254, and when an ultrasonic signal is output from the second ultrasonic sensor 254, the ultrasonic signal may be received by every ultrasonic sensor through plural paths.

FIG. 11 illustrates the paths and signal graphs of the direct signal and reflection signal output from the first ultrasonic sensor 252 and received by the third ultrasonic sensor 256 when the wearing angle of the wearable device 200 is changed.

According to an embodiment, the wearable device 200 may recognize, among the wave data of the ultrasonic signal received by the third ultrasonic sensor 256, a signal having an amplitude greater than or equal to a first reference value as a first ultrasonic signal (or, direct signal), and recognize a signal having an amplitude less than a second reference value being lower than the first reference value as a second ultrasonic signal (or, reflection signal). Since a portion of the ultrasonic signal passes through the bone 410 and only the remaining portion is reflected by the bone 410, the intensity (or amplitude) of the reflection signal detected by the third ultrasonic sensor 256 may be less than the intensity of the direct signal.

According to an embodiment, since the position of each ultrasonic sensor in the wearable device 200 is fixed, the length of the direct path may always be the same. With reference to parts (a) and (b) of FIG. 11, even if the wearing angle of the wearable device 200 changes, the arrival time of the direct signal at the third ultrasonic sensor 256 may remain substantially the same. According to an embodiment, the direct signal is suitable for measuring the transmissive signal inside the human body due to the fixed position of the sensor, and thus it may be used as an indicator for measuring the ultrasound velocity inside the human body.

According to an embodiment, the reflection signal, which is output from the first ultrasonic sensor 252, reflected by the bone 410, and received by the third ultrasonic sensor 256, may have a travel distance that varies depending on the wearing angle of the wearable device 200. Comparing part (a) and part (b) of FIG. 11, the length of the reflected path may be shorter in part (b) than in part (a) as the wearing angle of the wearable device 200 changes. Consequently, the time it takes for the reflection signal to reach the third ultrasonic sensor 256 may be earlier in part (b) than in part (a).

According to an embodiment, even if the wearing angle of the wearable device 200 changes, the ratio of the arrival times (or travel times) of the direct and reflected signals at a specific temperature may remain the same. For example, with reference to the graph in part (a) of FIG. 11, for ultrasonic signals measured in three different temperature states, the ratio between the arrival time of the direct signal and the arrival time of the reflection signal may be the same. In addition, even if the wearing angle is changed as in part (b) in FIG. 11, for ultrasonic signals measured in different temperature states, the ratio between the arrival time of the direct signal and the arrival time of the reflection signal may be the same.

According to an embodiment, the wearable device 200 (or, portable device) may predict the current wearing state (e.g., wearing angle) of the wearable device 200 based on the ratio of the arrival times (or, travel times) of the direct signal and the reflection signal.

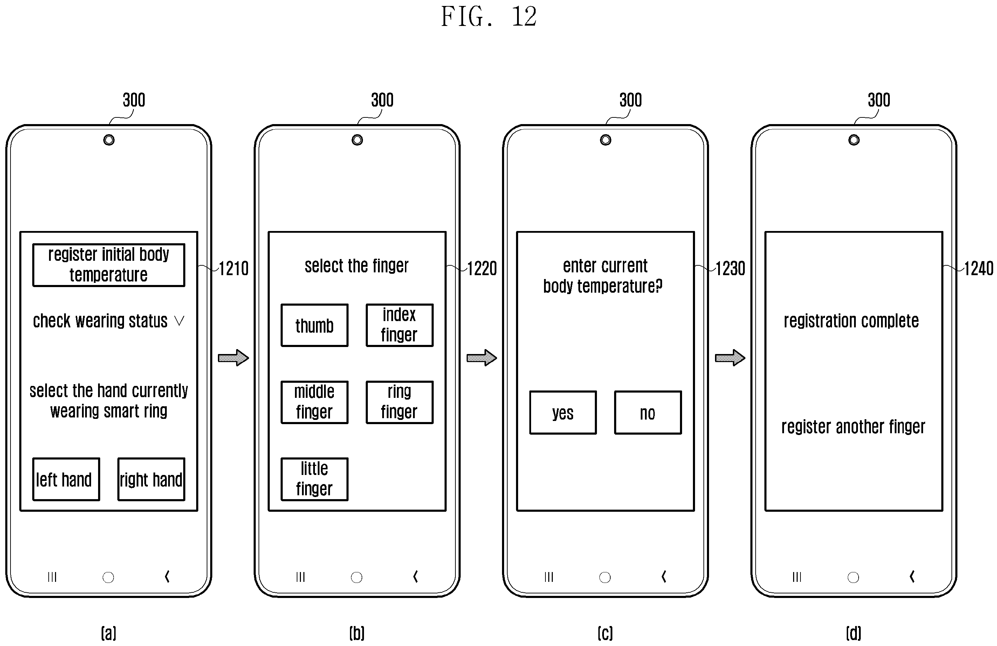

FIG. 12 illustrates a UI provided by the portable device according to an embodiment.

According to an embodiment, the portable device 300 may install and execute a healthcare application that provides various biometric information measured by the wearable device and provides a UI that allows the user to enter data related to the measurement of biometric information.

According to an embodiment, the portable device 300 may provide, through the display, a user interface (UI) that allows the user to enter the current body temperature on the healthcare application.

With reference to part (a) of FIG. 12, the portable device 300 may execute a menu for registering the initial body temperature and provide a UI 1210 for selecting one of the right hand and the left hand currently wearing the wearable device.

With reference to part (b) of FIG. 12, when the wearing hand is selected, the portable device 300 may provide a UI 1220 that allows the user to select one of thumb, index finger, middle finger, ring finger, and little finger, on which the wearable device is worn.

With reference to part (c) of FIG. 12, when the hand and finger wearing the wearable device are selected, the portable device 300 may provide a UI 1230 that identifies whether to input the current body temperature.

With reference to part (d) of FIG. 12, when current body temperature information is input according to user input, the portable device 300 may store the body temperature information in the memory and provide a UI 1240 that indicates completion of registration.

According to an embodiment, when user's body temperature information is input through the UI, the wearable device (or, portable device 300) may establish and store a mapping between the current wearing state (e.g., wearing position, angle) of the wearable device and the input body temperature information. According to an embodiment, the portable device 300 may provide a UI that allows the user to change the wearing state of the wearable device. In response to the UI, when the user changes the wearing position or angle of the wearable device, the portable device 300 may then measure the movement velocity of the ultrasonic signal by using ultrasonic sensors. Thereby, the wearable device and the portable device 300 may establish and store a mapping between the movement velocity of the ultrasonic signal and body temperature information in various wearing states.

According to an embodiment, in addition to body temperature information, the portable device 300 may further provide a UI for inputting other user body information (e.g., body fat percentage, skeletal muscle mass, BMI index) that may affect the velocity of the ultrasonic signal. The portable device 300 (or, wearable device) may correct the table or equation that maps the movement velocity of the ultrasonic wave and body temperature based on the body information input through the UI.

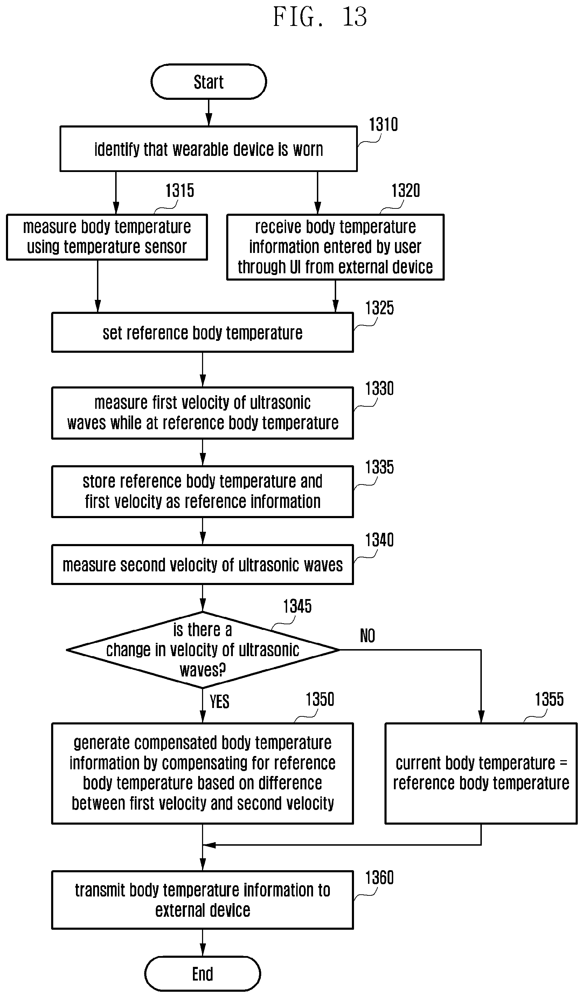

FIG. 13 is a flowchart of a method for measuring body temperature using ultrasonic waves in the wearable device according to an embodiment.

The method illustrated in FIG. 13 may be performed by the wearable device (e.g., wearable device 200 in FIGS. 3A and 3B), and the description of the technical features described above may be omitted below.

In the following embodiment, the operations may be performed sequentially, but are not necessarily performed sequentially. For example, the order of operations may be changed, and at least two operations may be performed in parallel.

According to an embodiment, operations 1310 to 1360 may be understood to be executed by the processor (e.g., processor 210 in FIG. 3A) of the wearable device (e.g., wearable device 200 in FIGS. 3A and 3B).

According to an embodiment, at operation 1310, the wearable device may identify that the wearable device is worn on a user's finger. The wearable device may be formed to enclose an empty central hole to be worn on the user's finger, and at least one sensor (e.g., biometric sensor, temperature sensor, ultrasonic sensor) for obtaining user's biometric information may be disposed in a direction toward the central hole within the housing.

According to an embodiment, at operation 1315, the wearable device may measure the user's current body temperature by using a temperature sensor. The temperature sensor may measure the user's skin temperature by utilizing an optical signal (e.g., infrared signal) based on the Stefan-Boltzmann law.

According to an embodiment, at operation 1320, the wearable device may receive body temperature information entered by the user through a UI from an external device (e.g., portable device 300 in FIG. 4). The UI for entering user's body temperature information on a healthcare application running on the external device is described in relation to FIG. 12.

According to an embodiment, at operation 1325, the wearable device may set the user's current body temperature measured using the temperature sensor and/or body temperature information received from the external device as the reference body temperature.

According to an embodiment, at operation 1330, the wearable device may measure a first velocity of the ultrasonic signal by using at least one ultrasonic sensor while at the reference body temperature. For example, in response to obtaining body temperature information through the temperature sensor and/or receiving body temperature information from the portable device, the wearable device may measure the first velocity of ultrasonic waves by using ultrasonic sensors. That is, the first velocity of ultrasonic waves may be measured at the reference body temperature.

According to an embodiment, at operation 1335, the wearable device may store the set reference body temperature and the measured first velocity in the memory as reference information. According to an embodiment, when measuring the first velocity (or, reference velocity), the wearable device may determine the first velocity based on the movement velocities obtained respectively on the movement paths of plural ultrasonic signals.

According to an embodiment, at operation 1340, after setting the reference information, when a body temperature measurement event occurs, the wearable device may measure the second velocity of ultrasonic signals by using at least one ultrasonic sensor. For example, the wearable device may output an ultrasonic signal to measure the second velocity of the ultrasonic signal periodically after storing the reference information, and/or when the user checks their current body temperature by using a healthcare application on the portable device. According to an embodiment, when measuring the second velocity, the wearable device may determine the second velocity based on an average (or weighted average) of the movement velocities of direct signals and reflection signals obtained respectively on the movement paths of plural ultrasonic signals.

According to an embodiment, at operation 1345, the wearable device may determine whether the second velocity of the ultrasonic signal is different from the first velocity of the ultrasonic signal stored as the reference information. Since the travel velocity of the ultrasonic signal may change with a temperature change of the medium, if the second velocity is different from the first velocity, it can be seen that the user's body temperature has changed.

According to an embodiment, if there is a change in the movement velocity of the ultrasonic signal, at operation 1350, the wearable device may compensate for the reference body temperature based on the difference between the first velocity and the second velocity to thereby generate compensated body temperature information. For example, in the wearable device, if the second velocity of the ultrasonic signal is measured to be faster than the first velocity, the reference body temperature may be compensated by a higher value; if the second velocity of the ultrasonic signal is measured to be slower than the first velocity, the reference body temperature may be compensated by a lower value; and the greater the difference between the first velocity and the second velocity, the larger the compensated body temperature value can be.

According to an embodiment, if there is no change in the movement velocity of the ultrasonic signal, at operation 1355, the wearable device may determine that the user's current body temperature is equal to the stored reference body temperature.

According to an embodiment, at operation 1360, the wearable device may transmit the body temperature information determined at operation 1350 or 1355 to the external device. The external device may provide the received body temperature information to the user through a UI.

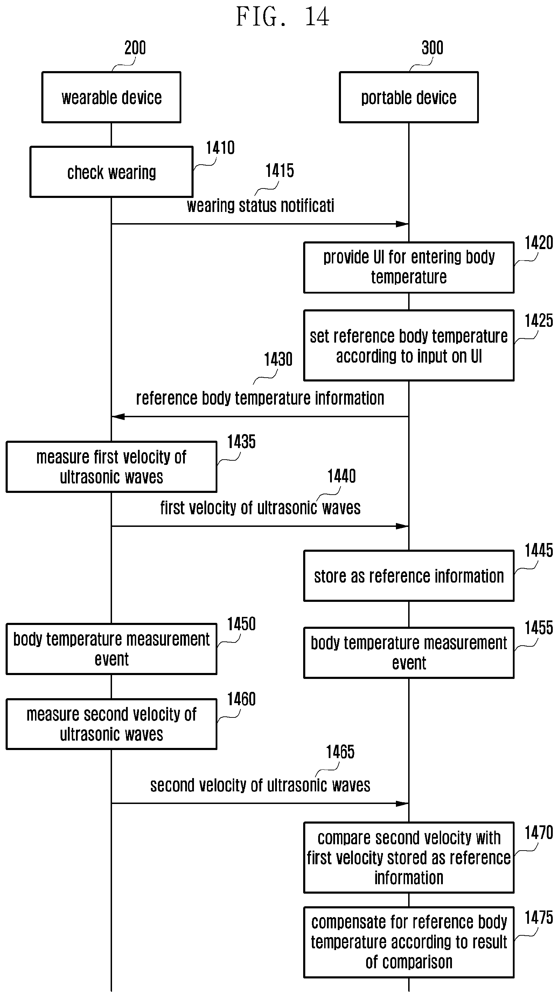

FIG. 14 is a flow diagram of a method for measuring body temperature using ultrasonic waves in a wearable device and a portable device according to an embodiment.

The method illustrated in FIG. 14 may be performed by the wearable device (e.g., wearable device 200 in FIGS. 3A and 3B) and the portable device (e.g., portable device 300 in FIG. 4), and the description of the technical features described above may be omitted below.

In the following embodiment, the operations may be performed sequentially, but are not necessarily performed sequentially. For example, the order of operations may be changed, and at least two operations may be performed in parallel.

According to an embodiment, operations 1410 to 1475 may be understood to be performed by the processor (e.g., processor 210 in FIG. 3A) of the wearable device (e.g., wearable device 200 in FIGS. 3A and 3B) or the processor (e.g., processor 310 in FIG. 4) of the portable device (e.g., portable device 300 in FIG. 4).

According to an embodiment, at operation 1410, the wearable device may identify that the wearable device is worn on a user's finger. At operation 1415, the wearable device may transmit its wearing status to the portable device. According to an embodiment, the wearable device may be connected to the portable device via short-range wireless communication (e.g., Bluetooth, Wi-Fi), and may transmit various data related to whether the wearable device is being worn, its current status, and obtained biometric information to the portable device.

According to an embodiment, at operation 1420, if the wearable device is confirmed to be worn, the portable device may execute a healthcare application and provide a UI for entering the user's body temperature via the display. At operation 1425, the portable device may set the reference body temperature based on user input on the UI. At operation 1430, the portable device may transmit the set reference body temperature information to the wearable device.

According to an embodiment, at operation 1435, the wearable device may measure the first velocity of the ultrasonic signal by using at least one ultrasonic sensor. The wearable device may include plural ultrasonic sensors disposed to be spaced apart from each other, and may determine the first velocity of the ultrasonic signal by sequentially outputting an ultrasonic signal from each of the ultrasonic sensors and using the movement velocities of ultrasonic signals received by all ultrasonic sensors along various paths (e.g., direct path, reflected path).

According to an embodiment, at operation 1440, the wearable device may transmit the measured first velocity of the ultrasonic signal to the portable device. At operation 1445, the wearable device may store the reference body temperature and the first velocity as reference information in the memory.

According to an embodiment, at operation 1450, a body temperature measurement event may occur in the wearable device, or at operation 1455, a body temperature measurement event may occur in the portable device.

According to an embodiment, at operation 1460, the wearable device may measure the second velocity of the ultrasonic signal by using ultrasonic sensors. According to an embodiment, when measuring the second velocity, the wearable device may determine the second velocity based on an average (or weighted average) of the movement velocities of direct signals and reflection signals obtained respectively on the movement paths of plural ultrasonic signals. At operation 1465, the wearable device may transmit the measured second velocity to the portable device.

According to an embodiment, at operation 1470, the portable device may compare the second velocity of the ultrasonic signal with the first velocity stored as the reference information, and at operation 1475, may compensate for the reference body temperature based on the comparison result to generate compensated body temperature information. Since the travel velocity of the ultrasonic signal may change with a temperature change of the medium, if the second velocity is different from the first velocity, it can be seen that the user's body temperature has changed. In the portable device, if the second velocity of the ultrasonic signal is measured to be faster than the first velocity, the reference body temperature may be compensated by a higher value; if the second velocity of the ultrasonic signal is measured to be slower than the first velocity, the reference body temperature may be compensated by a lower value; and the greater the difference between the first velocity and the second velocity, the larger the compensated body temperature value can be.

An electronic device according to various embodiments of this document may include a temperature sensor for measuring the user's body temperature, a wireless communication circuit for supporting wireless communication with an external device, at least one ultrasonic sensor, a memory, and at least one processor operably connected to the temperature sensor, the wireless communication circuit, the at least one ultrasonic sensor, and the memory.

According to an embodiment, the memory may store instructions that are executable by the at least one processor and, when executed, cause the electronic device to: set a reference body temperature based on at least one of body temperature information measured by the temperature sensor or body temperature information received through the wireless communication circuit from the external device; measure a first velocity of ultrasonic waves passing through the user's body by using the at least one ultrasonic sensor while the electronic device is worn by the user; and store the measured first velocity of ultrasonic waves and the set reference body temperature in the memory as reference information.

According to an embodiment, the memory may store instructions that cause the electronic device to: measure, after storing the reference information, a second velocity of ultrasonic signals passing through the user's body by using the at least one ultrasonic sensor; and generate compensated body temperature information by compensating for the reference body temperature based on a difference between the measured first velocity and the second velocity.

According to an embodiment, the memory may store instructions that cause the electronic device to generate the compensated body temperature information by compensating for the reference body temperature by a higher value if the second velocity is higher than the first velocity.

According to an embodiment, the at least one ultrasonic sensor may include a first ultrasonic sensor and a second ultrasonic sensor that are disposed to be spaced apart from each other; the second ultrasonic sensor may detect a first ultrasonic signal that is output from the first ultrasonic sensor and received through a direct path between the first ultrasonic sensor and the second ultrasonic sensor; and the processor may be configured to generate the compensated body temperature information based on the velocity of the first ultrasonic signal.

According to an embodiment, the second ultrasonic sensor may further detect a second ultrasonic signal that is output from the first ultrasonic sensor, reflected by a part of the user's body, and received through a reflected path; and the memory may store instructions that cause the electronic device to generate the compensated body temperature information further based on the velocity of the second ultrasonic signal.

According to an embodiment, the memory may store instructions that cause the electronic device to recognize, among ultrasonic data received by the second ultrasonic sensor, a signal having an amplitude greater than or equal to a first reference value as the first ultrasonic signal, and to recognize a signal having an amplitude less than a second reference value lower than the first reference value as the second ultrasonic signal.

According to an embodiment, the memory may store instructions that cause the electronic device to determine an average value of the movement velocity of the first ultrasonic signal and the movement velocity of the second ultrasonic signal to be the second velocity.

According to an embodiment, the memory may store instructions that cause the electronic device to control the at least one ultrasonic sensor to output ultrasonic waves at a sampling rate greater than or equal to a reference to measure the second velocity of ultrasonic waves.

According to an embodiment, the temperature sensor may be configured to measure the body temperature information by using a different type of signal from the ultrasonic sensor.

According to an embodiment, the electronic device may further include a ring-shaped housing including an empty central hole, and the at least one ultrasonic sensor may be disposed in a direction toward the central hole in the housing.

A method for an electronic device to measure the body temperature using ultrasonic waves according to various embodiments of this document may include: setting a reference body temperature based on at least one of body temperature information measured by a temperature sensor or body temperature information received from an external device; measuring a first velocity of ultrasonic waves passing through the user's body by using at least one ultrasonic sensor while the electronic device is worn by the user; storing the measured first velocity of ultrasonic waves and the set reference body temperature in a memory as reference information; measuring, after storing the reference information, a second velocity of ultrasonic signals passing through the user's body by using the at least one ultrasonic sensor; and generating compensated body temperature information by compensating for the reference body temperature based on a difference between the measured first velocity and the second velocity.

According to an embodiment, generating compensated body temperature information may include compensating for the reference body temperature by a higher value if the second velocity is higher than the first velocity.

According to an embodiment, the at least one ultrasonic sensor may include a first ultrasonic sensor and a second ultrasonic sensor that are disposed to be spaced apart from each other; the second ultrasonic sensor may detect a first ultrasonic signal that is output from the first ultrasonic sensor and received through a direct path between the first ultrasonic sensor and the second ultrasonic sensor; and generating compensated body temperature information may include generating the compensated body temperature information based on the velocity of the first ultrasonic signal.

According to an embodiment, the second ultrasonic sensor may further detect a second ultrasonic signal that is output from the first ultrasonic sensor, reflected by a part of the user's body, and received through a reflected path; and generating compensated body temperature information may include generating the compensated body temperature information further based on the velocity of the second ultrasonic signal.

According to an embodiment, the method may include recognizing, among ultrasonic data received by the second ultrasonic sensor, a signal having an amplitude greater than or equal to a first reference value as the first ultrasonic signal, and recognizing a signal having an amplitude less than a second reference value lower than the first reference value as the second ultrasonic signal.

According to an embodiment, measuring a second velocity of ultrasonic signals may include determining an average value of the movement velocity of the first ultrasonic signal and the movement velocity of the second ultrasonic signal to be the second velocity.

According to an embodiment, it is possible to control the at least one ultrasonic sensor to output ultrasonic waves at a sampling rate greater than or equal to a reference to measure the second velocity of ultrasonic waves.

An electronic device according to various embodiments of this document may include a display, a wireless communication circuit for supporting wireless communication with an external device, a memory, and at least one processor operably connected to the display, the wireless communication circuit, and the memory, wherein the memory may store instructions that are executable by the at least one processor and, when executed, cause the electronic device to: set a reference body temperature based on at least one of a user input through the display or body temperature information received through the wireless communication circuit from the external device; receive, through the wireless communication circuit, a first velocity of ultrasonic waves passing through the user's body, measured by the external device using at least one ultrasonic sensor; store the measured first speed of ultrasonic waves and the set reference body temperature as reference information in the memory; receive, after storing the reference information, through the wireless communication circuit, a second velocity of ultrasonic waves passing through the user's body, measured by the external device using the at least one ultrasonic sensor; and generate compensated body temperature information by compensating for the reference body temperature based on a difference between the received first velocity and the second velocity.

An electronic device according to various embodiments of this document may include a first ultrasonic sensor, a second ultrasonic sensor disposed to be spaced apart from the first ultrasonic sensor, a memory, and a processor operably connected to the first ultrasonic sensor, the second ultrasonic sensor, and the memory, wherein the memory may store a mapping table that maps the movement velocity of an ultrasonic signal and the user's body temperature for each movement path of the ultrasonic signal, wherein the memory may store instructions that are executable by at least one processor and, when executed, cause the electronic device to: identify the movement velocity of a first ultrasonic signal that is output from the first ultrasonic sensor and received through a direct path between the first ultrasonic sensor and the second ultrasonic sensor; identify the movement velocity of a second ultrasonic signal that is output from the first ultrasonic sensor, reflected by a part of the user's body, and received through a reflected path; calculate a weighted average of the movement velocity of the first ultrasonic signal and the movement velocity of the second ultrasonic signal; obtain, from the mapping table stored in the memory, body temperature information corresponding to the weighted average of the movement velocity of the first ultrasonic signal and the movement velocity of the second ultrasonic signal, and determine the body temperature information obtained from the mapping table to be the user's current body temperature.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or plural entities, and some of the plural entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device comprising:
a temperature sensor for measuring a user's body temperature;
a wireless communication circuit for supporting wireless communication with an external device;
at least one ultrasonic sensor;
memory; and
at least one processor operably connected to the temperature sensor, the wireless communication circuit, the at least one ultrasonic sensor, and the memory,
wherein the memory stores instructions that are executable by the at least one processor and, when executed, cause the electronic device to:
configure a reference body temperature based on at least one of body temperature information measured by the temperature sensor or body temperature information received through the wireless communication circuit from the external device;
measure a first velocity of ultrasonic waves passing through a user's body by using the at least one ultrasonic sensor while the electronic device is worn by a user;
store the measured first velocity of ultrasonic waves and the configured reference body temperature in the memory as reference information;
measure, after storing the reference information, a second velocity of ultrasonic signals passing through the user's body by using the at least one ultrasonic sensor; and
generate compensated body temperature information by compensating for the reference body temperature based on a difference between the measured first velocity and the second velocity.

2. The electronic device of claim 1, wherein the memory stores instructions that cause the electronic device to generate the compensated body temperature information by compensating for the reference body temperature by a higher value in case that the second velocity is higher than the first velocity.

3. The electronic device of claim 1 or 2, wherein:
the at least one ultrasonic sensor includes a first ultrasonic sensor and a second ultrasonic sensor that are disposed to be spaced apart from each other;
the second ultrasonic sensor detects a first ultrasonic signal that is output from the first ultrasonic sensor and received through a direct path between the first ultrasonic sensor and the second ultrasonic sensor; and
the memory stores instructions that cause the electronic device to generate the compensated body temperature information based on a velocity of the first ultrasonic signal.

4. The electronic device of claim 3, wherein:
the second ultrasonic sensor further detects a second ultrasonic signal that is output from the first ultrasonic sensor, reflected by a part of the user's body, and received through a reflected path; and
the memory stores instructions that cause the electronic device to generate the compensated body temperature information further based on a velocity of the second ultrasonic signal.

5. The electronic device of claim 4, wherein the memory stores instructions that cause the electronic device to recognize, among ultrasonic data received by the second ultrasonic sensor, a signal having an amplitude greater than or equal to a first reference value as the first ultrasonic signal, and to recognize a signal having an amplitude less than a second reference value lower than the first reference value as the second ultrasonic signal.

6. The electronic device of claim 4, wherein the memory stores instructions that cause the electronic device to determine an average value of the movement velocity of the first ultrasonic signal and the movement velocity of the second ultrasonic signal to be the second velocity.

7. The electronic device of any one of claims 1 to 6, wherein the memory stores instructions that cause the electronic device to control the at least one ultrasonic sensor to output ultrasonic waves at a sampling rate greater than or equal to a reference to measure the second velocity of ultrasonic waves.

8. The electronic device of any one of claims 1 to 7, wherein the temperature sensor is configured to measure the body temperature information by using a different type of signal from the ultrasonic sensor.

9. The electronic device of any one of claims 1 to 8, further comprising a ring-shaped housing including an empty central hole, and wherein the at least one ultrasonic sensor is disposed in a direction toward the central hole in the housing.

10. A method for an electronic device to measure a body temperature using ultrasonic waves, the method comprising:
configuring a reference body temperature based on at least one of body temperature information measured by a temperature sensor or body temperature information received from an external device;
measuring a first velocity of ultrasonic waves passing through a user's body by using at least one ultrasonic sensor while the electronic device is worn by the user;
storing the measured first velocity of ultrasonic waves and the configured reference body temperature in a memory as reference information;
measuring, after storing the reference information, a second velocity of ultrasonic signals passing through the user's body by using the at least one ultrasonic sensor; and
generating compensated body temperature information by compensating for the reference body temperature based on a difference between the measured first velocity and the second velocity.

11. The method of claim 10, wherein generating compensated body temperature information comprises compensating for the reference body temperature by a higher value in case that the second velocity is higher than the first velocity.

12. The method of claim 10 or 11, wherein:
the at least one ultrasonic sensor includes a first ultrasonic sensor and a second ultrasonic sensor that are disposed to be spaced apart from each other;
the second ultrasonic sensor detects a first ultrasonic signal that is output from the first ultrasonic sensor and received through a direct path between the first ultrasonic sensor and the second ultrasonic sensor; and
generating compensated body temperature information comprises generating the compensated body temperature information based on a velocity of the first ultrasonic signal.

13. The method of claim 12, wherein:
the second ultrasonic sensor further detects a second ultrasonic signal that is output from the first ultrasonic sensor, reflected by a part of the user's body, and received through a reflected path; and
generating compensated body temperature information comprises generating the compensated body temperature information further based on a velocity of the second ultrasonic signal.

14. The method of claim 13, comprising recognizing, among ultrasonic data received by the second ultrasonic sensor, a signal having an amplitude greater than or equal to a first reference value as the first ultrasonic signal, and recognizing a signal having an amplitude less than a second reference value lower than the first reference value as the second ultrasonic signal.

15. An electronic device comprising:
a display;
a wireless communication circuit for supporting wireless communication with an external device;
a memory; and
at least one processor operably connected to the display, the wireless communication circuit, and the memory,
wherein the memory stores instructions that are executable by the at least one processor and, when executed, cause the electronic device to:
configure a reference body temperature based on at least one of a user input through the display or body temperature information received through the wireless communication circuit from the external device;
receive, through the wireless communication circuit, a first velocity of ultrasonic waves passing through a user's body, measured by the external device using at least one ultrasonic sensor;
store the measured first speed of ultrasonic waves and the configured reference body temperature as reference information in the memory;
receive, after storing the reference information, through the wireless communication circuit, a second velocity of ultrasonic waves passing through the user's body, measured by the external device using the at least one ultrasonic sensor; and
generate compensated body temperature information by compensating for the reference body temperature based on a difference between the received first velocity and the second velocity.
